(19) Europäisches Patentamt / European Patent Office / Office européen des brevets

(11) **EP 3 244 902 B1**

(12) **EUROPEAN PATENT SPECIFICATION**

(45) Date of publication and mention of the grant of the patent:
**16.09.2020 Bulletin 2020/38**

(21) Application number: **16712714.1**

(22) Date of filing: **15.01.2016**

(51) Int Cl.:
*A61K 35/74* (2015.01)          *A61K 35/742* (2015.01)
*A61K 35/747* (2015.01)          *A61K 36/02* (2006.01)
*A61K 36/06* (2006.01)          *A61K 36/064* (2006.01)
*A61P 31/04* (2006.01)

(86) International application number:
**PCT/CZ2016/000007**

(87) International publication number:
**WO 2016/112881 (21.07.2016 Gazette 2016/29)**

(54) **DUAL MICROBIAL PREPARATION FOR LONG-TERM SUPPRESSION OR PREVENTION OF SYMPTOMS OF OPPORTUNISTIC MICROBIAL INFECTIONS**

DUALES MIKROBIELLES PRÄPARAT ZUR LANGFRISTIGEN UNTERDRÜCKUNG ODER VORBEUGUNG VON SYMPTOMEN VON OPPORTUNISTISCHEN MIKROBIELLEN INFEKTIONEN

DOUBLE PRÉPARATION MICROBIENNE POUR LA SUPPRESSION OU LA PRÉVENTION À LONG TERME DES SYMPTÔMES DES INFECTIONS MICROBIENNES OPPORTUNISTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **16.01.2015 CZ 20150023**

(43) Date of publication of application:
**22.11.2017 Bulletin 2017/47**

(73) Proprietor: **Bio Agens Research And Development - Bard, S.R.O.**
**160 00 Praha 6 (CZ)**

(72) Inventors:
• **SUCHÁNEK, Martin**
  **160 00 Praha 6 (CZ)**
• **KLIMES, Radim**
  **161 00 Praha 6 (CZ)**

(74) Representative: **Smrckova, Marie**
**Velflíkova 10**
**160 00 Praha 6 (CZ)**

(56) References cited:
**WO-A1-2011/054322          WO-A1-2014/147528**
**US-A1- 2013 101 576**

• **DATABASE WPI Week 201437 26 March 2014 (2014-03-26) Thomson Scientific, London, GB; AN 2014-J49949 XP002758190, & CN 103 651 582 A (ZHOU J) 26 March 2014 (2014-03-26)**
• **DATABASE EMBASE [Online] ELSEVIER SCIENCE PUBLISHERS, AMSTERDAM, NL; 1 January 2010 (2010-01-01), Sandikar BM et al: "Screening of antagonistic Bacillus species against phytopathogenic Pythium and Fusarium species", XP002758191, Database accession no. EMB-2010625002**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the Invention

The invention concerns a dual microbial preparation for the long-term suppression of symptoms of opportunistic microbial infections, in particular opportunistic microbial infections caused by dysbiosis (disruption of the microbial balance).

### Background of the Invention

The incidence of opportunistic microbial infections, inflamed, non-healing wounds, infections by aggressive forms of commensal yeasts and mycotic infections by dermatophytes has a rising tendency, and these problems now affect a significant segment of the population. This is primarily caused by the fact that an increase in the incidence of such illnesses is not normally caused by either outside epidemiological factors or by genetic defects which cause fundamental disruption of the physiological program of the immunity reactions of the host. The main cause of opportunistic microbial infections nowadays is temporary weakening of the immune system and of the natural defensive reactions, most commonly as a consequence of stress, demanding lifestyle, excessive strain on the organism in sportsmen, women or strenuous workers or in the elderly where it is associated with a natural weakening of immunity reactions. Also, it may be a consequence of the effects of serious accompanying illnesses and manifestations, such as diabetes, obesity, food allergies, autoimmune illnesses etc. A common and conspicuous accompanying manifestation in most opportunistic microbial infections is dysbiosis, meaning disruption of physiological balance in the composition of the healthy microbial flora characteristic for the individual parts of human body. The manifestations specified above therefore assume massive dimensions and affect the medical condition of the population more than traditional causes of illness (injuries, infections, genetic defects).

Existing treatment of opportunistic microbial infections proceeds without an understanding of the delicate balance within the organism, particularly from the long-term perspective. The frequent use of antibiotic, antimycotic and antiseptic substances carries the risk of burdening the organism with reactive chemical substances, which are moreover rarely free of toxic side effects. Further, there are the endocrine disrupting effects in azole antimycotics which disrupt the natural hormonal regulation of the organism. The ill-considered use of antibiotics, which is known and criticized, but nevertheless continually occurs, is a typical example of the non-physiological and non-causal approach to the treatment of opportunistic microbial infections, causing the prolonged disruption of the sensitive balance of natural microflora on the surface of the skin and various mucous surfaces. Antibiotics most commonly destroy the beneficial microbial components of natural microflora, having minimal effect on the pathogenic microorganisms, particularly on those pathogens capable of producing microbial biofilms. Their use over the long-term thereafter leads only to the creation of resistance due to antibiotics, antimycotics, antiseptics, meaning that these substances soon lose the rest of their potency. Resistance to the available antibiotics has taken on epidemic proportions and has become a reason for fears among specialized medical experts, and the general public alike. Opportunistic microbial infections are not most common among those illnesses which directly threaten life, but they do fundamentally worsen the quality of the life. The inadequacy of using chemical preparations in treating these infections is thereafter manifested in the frequent recurrence of these infections and their symptoms after prior, "successful" treatment. By this process, the frequency of recurring infection continually rises until the patient is trapped within a permanent cycle of infection, treatment, reinfection, treatment and so on.

Among the most important cases of opportunistic microbial infections are gingivitis and periodontal disease, microbial inflammation causing complications in non-healing wounds, opportunistic yeast infections that affect the membrane of the urogenital tract, dermatophytoses in humans and animals, and microbial infection complicated by the creation of a biofilm. As with all infectious diseases, all these illnesses incorporate powerful environmental influences. For this reason, the care for a clean work and living environment is now considered an integral part of modern, effective treatment of the infectious diseases.

Acute gingivitis and chronic periodontal disease are two stages of dysbiosis in the oral cavity that cause an inflammatory reaction of the immune system, leading to the destruction of periodontal structures and the weakening of the periodontium and eventually its complete loss [20]. In the aggressive environment of the oral cavity, pathogenic bacteria of the oral cavity, non-physiologically expanded under the conditions of dysbiosis, become part of polymicrobial communities, so-called biofilms, which allows them to withstand the defensive reactions of the immune system as well as antibiotics taken either locally or generally [30], [9], [4], [5], [27]. Modern studies have already identified with sufficient accuracy both the healthy oral cavity microbiome, characterized by the presence of a green microbial complex containing bacteria of the *Streptococcus, Prevotella, Veilonella, Gemella, Granulicatella, Eikenela* and *Cypnocytophaga* genus, and the shift from G+ aerobic bacteria to the G- anaerobic types *Prevotella intermedia, Fusobacterium nucleatum, Porphyromonas gingivalis, Tannerella forsythia* and *Treponema denticola* characterizing an orange and red microbial complex, the incidence of which is typical for advanced dysbioses [1], [11], 19]. Excessive reproduction of pathogenic bacteria in the oral cavity leads to, in particular, bleeding gums, which is characterized by the value of the PBI Index (Papilla Bleeding Index), and to a general deterioration in the condition of the teeth and their prognosis, characterized by the Community Periodontal Index of Treatment Needs (CPITN), according to the WHO [2], [25]. Existing approaches consisting of the use of local or general antibiotics, local disinfecting agents (chlorhexidine and others), or mere physical cleaning and whitening of the teeth, do not bring the expected long-term effects [5]. Alternative approaches consisting of the use of probiotics are not overly effective, which is mainly ascribed to the fact that the "protective" microbial components used to date were derived from normal microbiomes in environments of the human body other than the oral cavity [35].

Microbial inflammation of wounds is one of the critical complicating factors in treating varicose ulcers among those suffering from diabetes, as well as those not [15]. The social burden of this disease lies in its long-term consequences from the perspective of incapacity to work and social discrimination against sufferers, and, primarily, in its colossal spread, accounting for up to 980 thousand cases a year in the countries of the EU (European Union) alone. Also worth mentioning are the enormous costs of treatment, estimated at around EUR 6.5 billion in the countries of the EU [12]. The chronic course of varicose ulcers itself increases susceptibility to infections as a consequence of damage to the skin barrier, problems with circulation, and inhibition of the defensive reactions of the immune system [37]. The symptoms of clinical infection include, in particular, flaring, pain, the creation of edema, smell symptoms and purulent discharge, which could turn into life-threatening septic conditions [8]. A recent publication by the EWMA (European Wound Management Association) is highly critical towards the treatment of microbial infections using local antibiotics or antiseptics, and calls for new solutions. The number of innovative approaches, however, is extremely limited and 85 % of the global market in products to eliminate inflammation of non-healing wounds is still based on the use of silver products, known since antiquity, regardless of the detailed knowledge of the normal skin microbiome we now have [18], [13].

Our knowledge of the etiology of opportunistic infections by pathogenic yeasts on the skin and mucosal surfaces has recently risen exponentially, although this has unfortunately been manifested only to a limited extent in the design of new therapeutic modalities. The actual naming of the yeast *Candida albicans,* and its related species, as pathogenic yeasts is not entirely accurate. It is an organism that is abundantly present in the environment, where it lives as a commensal microorganism on the skin and mucosal surfaces of humans, and is predominantly harmless to healthy individuals. However, it is good at recognizing the weakening of immunity, albeit temporary, and begins acting aggressively towards immunocompromised individuals. At the same time, it uses individual virulent factors, whose molecular nature has been better understood since the 1990s, such as adhesion proteins responsible for strong bonding to the surface of the epithelial cells of the host, factors responsible for a change in morphology from single-cell to hyphal form, and hydrolytic enzymes exemplified by secreted aspartate proteases or phospholipases [6]. Other virulent factors have the nature of released soluble glucans, which suppress the cytokine signaling of monocytes and also inhibit specific immunity that is dependent on T-lymphocytes [29]. Candida (single-cell yeasts) might then pose a considerable threat to permanently immunosuppressed patients because in them they can even survive in circulation and cause deadly infections [14]. Oral and vagina! candida infections are, however, very common among individuals suffering only from slightly weakened immunity [21]. Knowledge of the natural mechanisms of Candida infection has only been used to a minimum until now. Antibodies against all the virulent factors mentioned above are found at the laboratory testing stage, as are the chemical inhibitors of secreted aspartate proteases. However, they have not yet achieved greater clinical use [10]. The mechanisms of immune response to the yeasts have been advancing, but none of their biological enemies have been identified so far [16].

As with Candida (yeast) infections, in dermatophytoses, also, the permanent weakening of the immune system can lead to system infections. The recent sequencing of the genomes of several dermatophytes opened the way to identifying their infectivity genes, and to develop molecular diagnostics [3]. Otherwise, however, the treatment of these diseases is still dependent mainly on the use of chemical antimycotics that are notorious for their side effects, and for the fact that they only temporarily suppress the infection.

Dermatophytoses are among fungal infections widespread in humans and animals alike. We classify them as zoonoses, since in humans the infection often originates in animals. Of course, transmission in reverse, from human to animal, is also possible. The risk of transmissions of this kind is also now rising, because mycotic infections do not affect only farmers, meaning breeders that come into direct contact with animals bred for food and horses [28], but also those living in cities, where the transmission of infection comes about through close contact with pets kept in the households, where they live in close daily contact with their owners [24]. It is frequently hard to establish infection in animals in the case of entirely healthy individuals. However, the incidence of such infections is relatively common among stressed pets with a weakened immune system, and among breeds having a hereditary predisposition to skin diseases. Such illnesses also occur in the form of secondary infections which significantly hinder the course of the primary illness. Dermatophytic infections are common among large breeds. Breakouts principally occur when quarantine is not maintained upon the arrival of new animals, and when zoohygiene conditions are not carefully observed among the breed. In the case of breeding farm animals, this can lead to significant economic losses. Zoonoses caused by pathogens of the *Trichophyton* genus must be reported to the competent authorities, this is mandatory. Treating these infections using classic chemical products is, therefore, a burden on the animals and on their owners. In animals whose products, such as meat and milk, reach the food chain, such treatment makes it impossible to process these raw materials within the set protective time limits so that the consumer is not put at risk by the residue of the therapeutics used.

The approach to the issue of opportunistic microbial infections only recently became focused and approached in a complex way, based on extensive large studies and good knowledge of their causes with a concomitant emphasis on effective natural methods associated with the so-called biological treatment. One fundamental benefit for the increasing popularity of biological approaches came in the form of the recent flow of results from the Human Microbiome Project Consortium, which, based on modern methods of molecular genetics, carried out a detailed analysis of individual physiological microbial communities occurring on the skin, on mucosal surfaces, and in the respiratory, digestive and urogenital tract [22]. Based on these internationally-verified and continually-supplemented analyses, the scientific and medical public first learned of the constitution of microbial communities in different parts of the human body, a fact only partly clarified to that time, based on a sufficient, statistically significant set of data from healthy individuals. This enabled an entirely new perspective of their individual variability, stability over time, and, in particular, changes in these communities as under the well characterized pathological conditions.

After publication of the results by the Human Microbiome Project Consortium, the scientific and medical public expected rapid use of this knowledge to promote new and effective treatment procedures, among other in the sphere of treating opportunistic microbial infections, whose link to microbial dysbiosis has been supported by a large amount of evidence. From the perspective of the above expectations, it can be commented that the number of practically tested treatment procedures which use the above mentioned revolutionary knowledge as well as the number of procedures based on any biological method of treatment remained far behind expectations. From the perspective of using biological methods of treating opportunistic infections, it is worth mentioning CZ 9883 U (18. 4. 2000) [36], which describes products to protect the skin using the oomycete *Pythium oligandrum* in the form of a biological preparation for subduing of the originators of dermatophytoses that contains, as its active component, the mentioned organism in the form of oospores while the total number of oospores is at the maximum of $2 \times 10^5$ per 1 g of the preparation.

The use of the *Pythium oligandrum* microorganism as a tool in the biological battle with fungal and other infections was then significantly broadened in CZ 302 297 (9. 2. 2011) [34], submitted and owned by the applicant. The invention describes an anti-fungal mixture containing the fungal organism *Pythium oligandrum,* intended to suppress human diseases and diseases in animals of fungal, bacterial or other origin and to disrupt biofilms on foreign materials used in human and veterinary medicine and to eliminate microflora from various items that come into contact with humans or animals. The anti-fungal mixture designed uses active substances of the *Pythium oligandrum* oomycete mixed with inert components, The activity of the *Pythium oligandrum* "fungal" organism comes about in the mixture at the moment when it comes into contact with moisture. The solution according to CZ 302 297 [34] is conceived in relation to the medical use of *Pythium oligandrum* oomycetes to suppress the symptoms of a range of different illnesses of diseases of fungal original. Based on this patent, its owner and, at the same time also the applicant, decided to develop, test and sell a range of cosmetic products. These products are on sale in pharmacies and from veterinarians in Czech Republic while notified at the relevant European cosmetics portal according to EU requirements. In relation to medical use, the low toxicity must be stressed, a fact presently supported by the experiences of a number of users on one hand, and by laboratory tests ordered for evaluation of the safety of a cosmetic product by the competent national on the other. Long-term use of this essentially mono-component preparation with active substance *Pythium oligandrum* has proved very successful. Nonetheless, it has been shown that it does not suit certain patients over the long-term. It has been shown that these are patients with weakened or otherwise disrupted immunity.

The use of biological preparations as protection against opportunistic infections is also the subject of several foreign patents and patent applications. US 5,190,746 (2.3.1993) [7] proposes the use of hexasaccharide of molecular mass 959 isolated from the oral microbe *Streplococcus oralis* to block mutual interaction of this streptococcus with *Capnocytophaga ochracea,* a component of bacterial dental plaque. In spite of the fact that the effect of oligosaccharide was only established in laboratory tests, it is assumed in the invention that it could be effective in the prevention, inhibition or disruption of microbial deposits in dental plaque, for example when used in a toothpaste or mouthwash. CA 2 374 938 A1 (30. 11.2001) [31] describes the method for inhibiting the infection of wounds in mammals consisting of applying probiotic organisms directly to the wound, whereby several strains of lactobacillus are proposed as the probiotic organism, for example *Lactobacillus rhamnosus, Lactobacillus acidophilus, Lactobacillus fermentum* etc. The same method is proposed to inhibit the formation of biofilms, not in non-healing wounds, but on various operation implements. WO 2008/ 077 251 A (3.7.2008) [32] deals with the use of lactobacilli to eliminate the methicillin-resistant pathogen *Staphylococcus aureus,* based on a laboratory test. WO 2013/122 931 A2 (22.8.2013) [26] describes the use of prebiotics supporting the development of healthy commensal organisms in the skin, namely the *Streptococcus epidermidis, Corynebacterium jeikeium* and *Propionibacterium* acnes bacteria with the aim to improve the physiological condition and appearance of the skin. The colonization of these microorganisms was proven using an *in vivo* test. However, there is no evidence in the study for the positive effect of microorganisms applied in this way to the skin or for their use in treatment.

Successful results were recorded in scientific and medical literature with the use of lactobacilli to increase female resistance to recurring vulvovaginal yeast infections. Particularly worth mentioning are the results achieved with the Lactin V preparation made by the company Osel in clinical trials sponsored by the University of Washington. The results of stages IIa and IIb of these studies, which are available in scientific publications [see ref. 33], showed that recurring infections of the urogenital tract occurred in 15 % of women treated with Lactin V, whereas the incidence was higher, around 27 %, in the group of untreated women. Colonization by the supplemented lactobacillus was proven using a genetic test, and a statistically significant correlation could be ascertained between the colonization identified in this way and a reduction in the incidence of recurring vaginal infections, and infections of the urogenital tract.

However, the inventions specified above have certain fundamental disadvantages. All the principles presented were only proven in laboratory tests or, at the very most, on animal experimental models. This shortcoming is fundamental as it is known to experts in the field that the results obtained on animal models need not always apply in the case of human treatment, and need not have universal application even in veterinary treatment (i.e. the findings made for one species of animal cannot always be effectively applied to another species of animal). Moreover, microorganisms without any physiological relevance to the area of treatment were used as probiotic organisms, most commonly lactobacilli, which constitute the predominant microbial component of the vaginal microbiome, but which practically do not physiologically occur in other microbiomes. Only in the case of the solution according to US 5,190,734 A [7] and WO 2013/122 931 A2 [26] were the components of natural, physiological microbiomes used in the oral cavity or in the skin, respectively. In these cases, however, these components involved prebiotics and not probiotics. Prebiotics are compounds which could, but do not necessarily have to, stimulate the growth and reproduction of the relevant probiotic organisms. In these cases, too, the objection stands that the effectiveness of these preparations was only proven in laboratory tests, and not in real situations in proven in clinical studies.

## The Summary of the Invention

The disadvantages specified above are eliminated or significantly restricted in a dual microbial preparation for the long-term suppression of symptoms or for the prevention of opportunistic microbial infections, in particular caused by dysbiosis, according to this invention. The essence of this invention lies in the fact that the dual-component microbial preparation contains two basic microbial components, the microscopic oomycete *Pythium oligandrum* and components of the microbiome, whereby the physiological microbiome component is a component of a microbiome of human or animal origin. The microscopic oomycete is present in the form of propagules and various released substances of macromolecular and low-molecular nature as one inseparable component.

The second component is a bacteria found in the physiological microbiome verified in clinical studies, supplied into the dual microbial preparation until the establishment of the required microbial balance in the human or animal organism. The physiological microbiome component is of human or animal origin. A physiological microbiome component of human origin is used to eliminate and prevent symptoms caused by dysbiosis in humans, whereby it is essential that the topological localization of this dysbiosis is taken into consideration. For example, the use of components of the oral microbiome only comes under consideration for the oral cavity. A physiological microbiome component of animal origin is used to eliminate and prevent symptoms caused by dysbiosis in animals, whereby it is essential that the topological localization of this dysbiosis is taken into consideration. For example, the use of components of the skin microbiome only comes under consideration for infection by dermatophytes on the skin or in the fur of the animals.

The main benefit of this invention is that the dual microbial preparation brings about a condition of continual and long-term stabilization of the healthy microbiome in the area of application. The major advantage of this solution is that both basic components of the microbial dual preparation act mutually with a strengthening and mutually acting (synergic) mechanism, together with the immune system of the host to eliminate the agents and circumstances of certain pathological conditions. Following the elimination of the pathological noxa facilitated by the unique activities of *Pythium oligandrum*, this oomycete remains in idle state in the area of application, whereas the second microbial component strengthens the weakened physiological components of the normal, healthy microbiome, and, through its metabolic processes, stabilizes and establishes the required healthy microenvironment in the area of application.

One extremely important advantage is the long-term positive effect of dual microbial preparations according to this invention, which distinguishes it from alternative chemical preparations that only usually have a short-term effect. This short-term effect can only be compensated by repeated use of the chemical preparations, during which, however, the target pathogenic organisms begin to exhibit resistance to these chemical compounds. The only way of overcoming this resistance is to use progressively ever higher doses of chemical preparations, which obviously multiplies any side effects and the toxic effects of these chemicals. The use of biological preparations has the undoubted advantage of using entirely different mechanisms which act more compatibly with the elimination of pathogenic infection and long-term stabilizing effects.

In the specific case of the proposed dual microbial preparation according to this invention, the results show that each of the two microorganisms used exhibit their own population curve that arises from their biological properties in the area of application. One component, *Pythium oligandrum,* shows a rapid increase at the initial stage depending on the presence of potential fungal or yeast nutrients, and, eventually, survives in the area of application in sporulated form after cleaning the area of application and suppressing the pathogens. The second, stabilizing component of the normal, healthy microbiome survives in the area of application for prolonged time, and strengthens the positive effects of the already present normal microflora helping in its stabilization.

The dual microbial preparation according to this invention contributes toward suppressing and eliminating opportunistic fungal, yeast and bacterial infections in humans and animals and creation of the healthy microbiome in the oral cavity and on the surface of non-healing wounds and at mucosal surfaces affected by yeast infections disrupted as a consequence of stress, an unhealthy lifestyle, physical exertion or overuse of chemical preparations in the personal hygiene.

The invention submitted is primarily suitable for suppressing a number of pathological symptoms based on stabilizing normal, healthy physiological microbiomes, whose natural balance is disrupted in the event of stress, excessive physical load, weakening of the organism, genetic disorders or other illnesses. The invention submitted is based on the synergic action of the microscopic oomycete *Pythium oligandrum,* whose primary role it is to clean the application micro-environment, eliminate fungal or yeast burden, disrupt the microbial biofilm in the area of application and suppress the growth of pathogenic bacteria and the dominant microbial component of the healthy physiological microbiome.

It is beneficial when the microscopic oomycete *Pythium oligandrum* and the physiological microbiome component are present in a form which facilitates their germination, subsequent propagation and colonization of the target tissue, in which they apply their normal metabolic activities depending on the local conditions, including the ability to release into their surroundings biologically active macromolecular or low-molecular substances. At the same time, physiological regulatory mechanisms, together with the limited surface capacity of the skin and the mucosal membranes, effectively prevent the possibility of supplying an excessive amount of the physiological microbiome component, since in such case the surface structural capacity is rapidly saturated and surplus components are automatically removed.

The dual microbial preparation according to this invention optimally contains an effective identified range of both fundamental components, meaning the microscopic oomycete *Pythium oligandrum* in a quantity of $10^3$ to $10^7$, preferentially $10^4$ to $10^5$, colony-forming units (CFU) per one gram of the preparation, in combination with the dominant component of the relevant microbiome in a quantity of $5 \times 10^6$ to $5 \times 10^{10}$, preferably $5 \times 10^7$ to $5 \times 10^9$, colony-forming units (CFU) per one gram of the preparation. It was identified that lower values of the claimed range of components are not sufficiently effective, whereas higher values could unnecessarily burden the human organism. At the same time, the ratio of both microbial components used appropriately draws on the mutual ratio of the biosynthetic capacities of both microorganisms used, which is essentially defined by the ratio of the volumes of both cell types used. It is, therefore, characterized by a conversion factor of approximately 2350, given the averaged size of the *Pythium oligandrum* oomycete of 18 x 18 x 18 $\mu$m and of bacteria of 1 x 1 x 2 $\mu$m.

A substrate fermented during the growth of the *Pythium oligandrum* oomycete could act as a source of nutrients for both microbial components in the dual microbial preparation according to this invention. Nutrients and prebiotics can potentially support the metabolism of both microbial components.

The optimum application of the dual microbial preparation according to this invention is supported by auxiliary components which contain at least one of the auxiliary components from each below described groups, comprising: a desiccant such as silicon dioxide; components of a buffer system such as citric acid, sodium bicarbonate and sodium carbonate, bringing about the right pH during application; an anti-caking substance such as sorbitol or polyethylene glycol; and an agent for the production of a physiological osmotic environment such as sodium chloride.

Specificity tests of mutual action under conditions close to those in different parts of the human body identified that the target microbial component of the relevant microbiome is invariably the dominant microbial component of the relevant microbiome, which is relevant under the conditions of the respective surface location.

For the long-term suppression of symptoms of periodontal illnesses and the long-term maintenance of a healthy oral cavity it is an advantage when the component of a healthy human microbiome is the component of the healthy oral microbiome, i.e. one of the microbes of the green complex, such as the *Capnocytophaga sputigena,* whereby its quantitative amount corresponds to the content of this component in the physiological microbiome.

Similarly, to ensure the long-term suppression of symptoms of clinical infection in non-healing wounds in non-diabetic patients, and the disruption of biofilms in non-healing wounds, including biofilms caused by the *Stenotrophomonas maltophila* and *Pseudomonas aeruginosa* microorganisms, and for the long-term maintenance of healthy skin, it is beneficial when a human microbiome component, i.e. one of the components of a healthy skin microbiome, such as the *Staphyloccocus epidermidis* bacterium, is used in the physiological amount.

In order to ensure the long-term suppression of yeast infections of the urogenital tract and skin and vaginal membrane, it is beneficial when a human microbiome component, i.e. one of the components of a healthy vaginal microbiome, such as peroxide-producing *Lactobacillus crispatus,* is the physiological microbiome component.

For cosmetic purposes or for patients with weakened immunity, it is beneficial when either the *Pythium oligandrum* microscopic oomycete or a physiological microbiome component of human or animal origin is present in the dual microbial preparation in the form of non-living components, such as dead cells, cell extracts or isolated cell fractionations,

It was identified that in order to eliminate the agents of mycotic diseases of the skin in animals and to ensure the long-term maintenance of healthy skin it is beneficial when a yeast autolysate in a quantity of 0.1 % - 10 % weight of the total quantity of the dual microbial preparation is used as non viable components increasing the effectiveness of the *Pythium oligandrum* oomycete.

To ensure easier application of the dual microbial preparation according to this invention, it is appropriate when auxiliary substances come in the form of ointments, oils, creams or suppositories or in the form of a liquid aqueous preparation.

With respect to the temperature growth limitation of the microscopic oomycete *Pythium oligandrum,* the temperature when applying the dual microbial preparation according to this invention should invariably be lower than the physiological temperature, such as a temperature of around 35 °C. This limitation practically precludes the use of dual microbial preparations according to this invention for internal use. For this reason, its use is limited to the skin and mucosal membranes of the oral cavity and urogenital tract, which it is possible to rinse with aqueous solutions of the proposed preparation at temperatures which are somewhat lower than the temperature of the human body.

When applied, the dual microbial preparation is activated in contact with moisture. The activity of the dual microbial preparation in the mixture comes about at the moment at which the preparation comes into contact with moisture or with wetted inert components. The application of a wetted, activated preparation is done mainly by rinsing the affected areas or other methods of wet application or the components can be provided in the form of ointments, creams, oils or suppositories, in the form of the dry lyophilized mixtures contained in these cosmetic products, or in the form of a liquid aqueous preparation.

Non-claimed comparative embodiments, for purpose of clarifying the invention, serves in order to suppress or disrupt biofilms which are resistant to the action of individual isolated components, when disrupting biofilms caused by microorganisms such as *Stenotrophomonas maltophila* or *Pseudomonas acruginosa,* and in order to eliminate fungi, yeasts and pathogenic bacteria from the environment, when the microbial component is one of the bacteria, in particular bacillus, capable of suppressing the development of fungi in the environment in a quantity of $10^5$ to $10^{12}$ colony-forming units (CFU) per one gram. The *Bacillus amyloliquefaciens* bacterium is preferentially the microbial component from the environment.

## Brief Description of the Drawings

The invention is further described in detail in Examples, and explained in more detail in the appended schematic drawings, in which

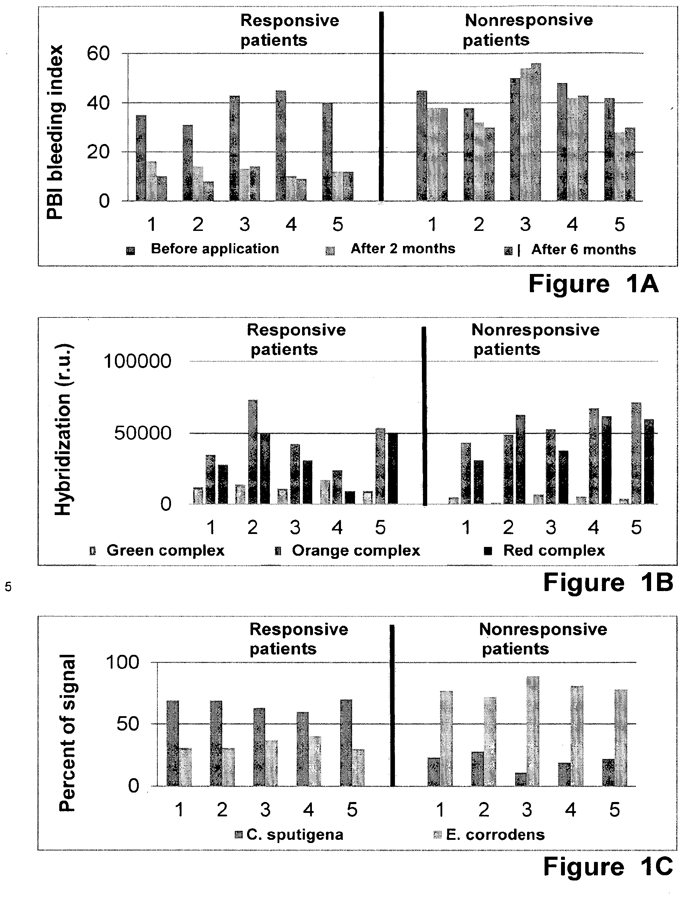

**Figure 1A, Figure 1B and Figure 1C** show the effectiveness test of the dual microbial preparation for the oral cavity for individual patients (numbered): for "responsive patients" on the left-hand side under conditions corresponding to the application of the dual component microbial preparation according to the invention; for "unresponsive" patients on the right-hand side under conditions corresponding to the application of a mono-component preparation based on *Pythium oligandrum* according to invention CZ 302 297 B6, and

**Figure 1A** in more detail the dependence of the PBI (Papilla Bleeding Index) of the gums before application, 2 months following application and 6 months following application;

**Figure 1B** shows the dependency of the intensity of hybridization in relative units (r.u.), corresponding to the number of disintegrations per minute (dpm) for the microorganisms pertaining to the specified microbial complexes;

**Figure 1C** shows the dependency of the content of bacteria of the green complex *Capnocytophaga sputigena* and *Eikenella corrodens* expressed as a percentage of the hybridization signals;

**Figure 2** shows the time stability of the component of the dual microbial preparation for treating symptoms of gingivitis (inflammation of the gums) and periodontal diseases in the oral cavity, showing the dependency of the number of colony-forming units (CFU) on the months of storage for *Pythium oligandrum* and for *Capnocytophaga sputigena,* respectively;

**Figure 3** shows test of the effectiveness of the dual preparation for non-healing wounds, shown are the percentages of the original condition: for "responsive patients" on the left-hand side under conditions corresponding to the application of the dual component microbial preparation according to the invention; and for "unresponsive" patients on the right-hand side under conditions corresponding to the application of a mono-component preparation based on *Pythium oligandrum* according to invention CZ 302 297 B6;

**Figure 4** shows time stability of the component of the dual microbial preparation for non-healing wounds, i.e. the dependency of the number of colonies on the months of storage for *Pythium oligandrum* and for *Staphylococcus epidermidis,* respectively;

**Figure 5A, Figure 5B, Figure 5C,** test of effectiveness of the dual preparation for a mucous membranes susceptible to the easts for individual patients: for "responsive patients" on the left-hand side under conditions corresponding to the application of the dual component microbial preparation according to the invention; and for "unresponsive" patients on the right-hand side under conditions corresponding to the application of a mono-component preparation based on *Pythium oligandrum* according to invention CZ 302 297 B6; whereby

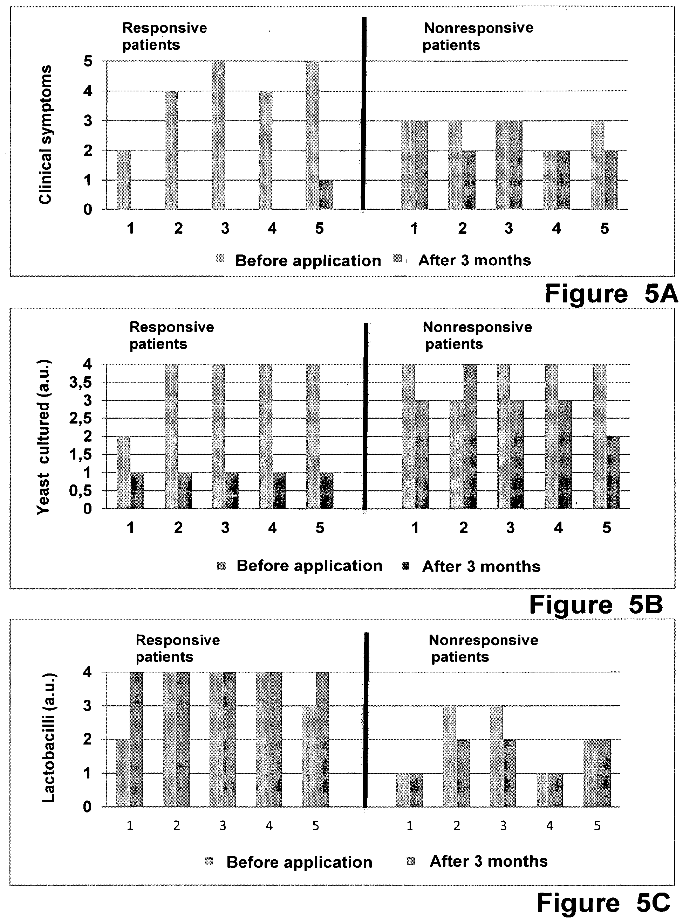

**Figure 5A** shows clinical evaluation, on a scale of 1 to 6, at the beginning before application and 3 months after application, where "1" indicates the elimination of one and 6 the elimination of all 6 symptoms monitored described as discomfort, irritation, burning, discharge, pain and reddening; and

**Figure 5B** shows the number of yeasts in arbitrary units, where "1" indicates incidence only following cultivation, "2" sporadic incidence, "3" frequent incidence, "4" massive incidence, all at the beginning before application and 3 months after application;

**Figure 5C** shows the number of *Lactobacillus* in the arbitrary units specified above, this at the beginning before application and 3 months after application;

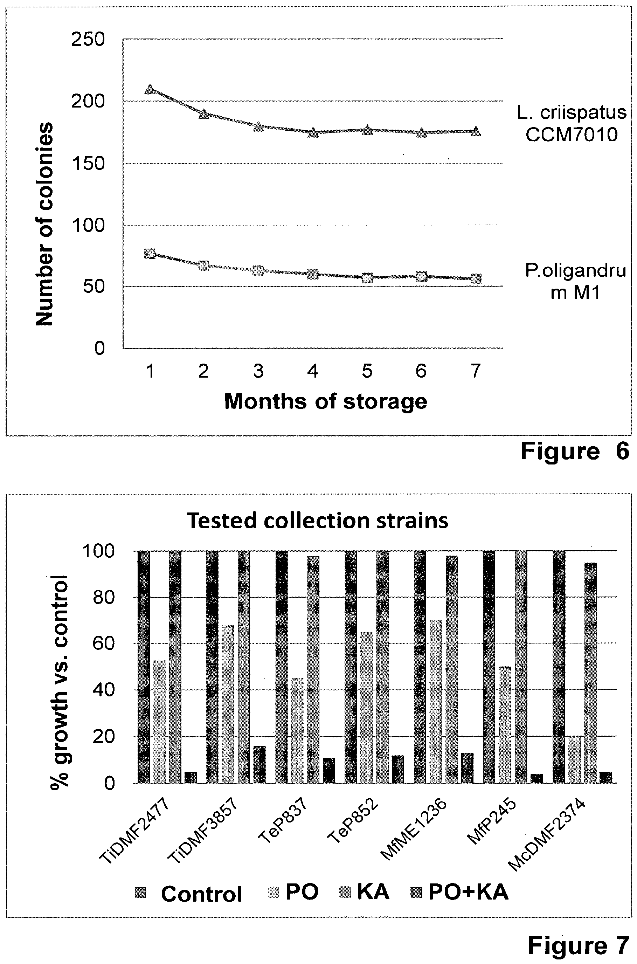

**Figure 6** shows the time stability of dual microbial preparations for skin and membranes susceptible to the propagation of pathogenic yeast cells, showing dependency of the number of colonies on months of storage for *Pythium oligandrum* and *Lactobacillus crispatus,* respectively;

**Figure 7** shows the *in vitro* effectiveness test for the dual microbial preparation for mycosis of the feet; showing the dependency of the number of colony-forming units (CFU) in % related to a placebo, where K is the control corresponding to the placebo, PO is *Pythium oligandrum,* KA is the yeast autolysate, PO + KA *Pythium oligandrum and yeast autolysate;* the tested strains of dermatophytes were the collection strains *Trichophyton interdigitale* DMF2477 (TiDMF2477), *Trichophyton interdigitale* DMF3857 (TiDMF3857), *Trichophyton erinacei* P837 (TeP837), *Trichophyton erinacei* P852 (TeP852), *Microsporum floccosum* ME1236 (MfME1236), *Microsporum floccosum* P245 (MfP245) and *Microsporum* canis DMF2374 (McDMF2374).

Non-claimed embodiments as comparative example schematicaly present

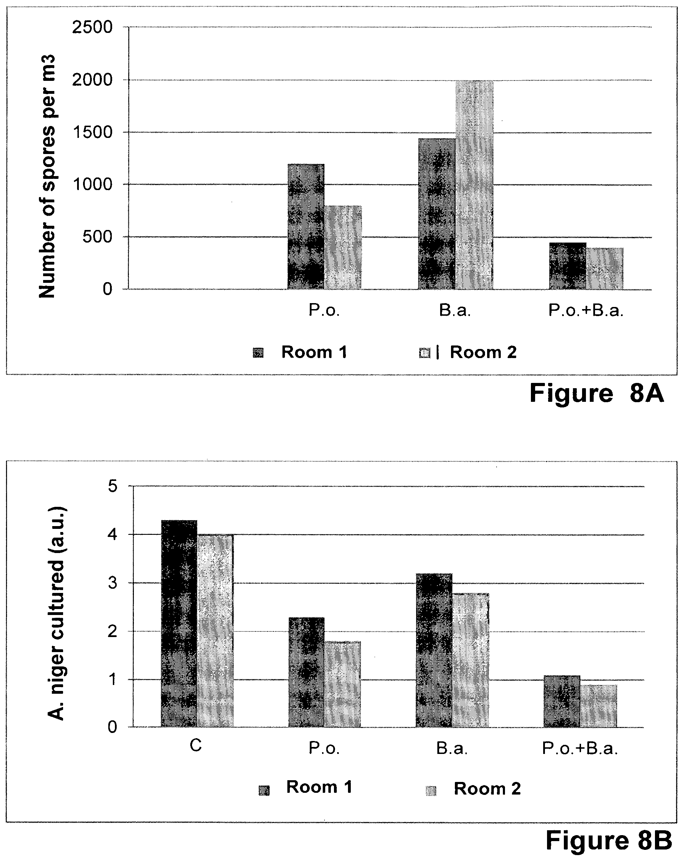

**Figure 8A, Figure 8B**, show the results of a test of the influence of the number of fungal spores in the atmosphere and test of contamination with funguses in scrapings in two different rooms with an incidence of fungus on the walls, with dominant incidence of the *Aspergillus niger* and *Cladosporium spp.* fungus, where CK are values corresponding to a control without any treatment, P. o. are values corresponding to the use of a mono-component preparation based on *Pythium oligandrum* according to invention CZ 302 297 B6, B. a. are values corresponding to the application of the *Bacillus amyloliquefaciens* bacillus alone and P.o.+B.a. are values corresponding to the application of a dual component microbial preparation according to the invention, where

**Figure 8A** shows the number of fungal spores per 1 m$^3$ of air in the contaminated rooms identified using the Omeljan method after collecting of fall-out on Petri dishes in that according to WHO standards this value should be below the limit value of 500;

**Figure 8B** shows the results of microbial samples and cultivation from scrapings expressed in arbitrary units on a scale of medical valuation of 1 to 4, where "1" indicates incidence only after cultivation, "2" sporadic incidence, "3" frequent incidence and "4" massive incidence.

## Description of the Preferred Embodiments

The solution which we propose is enabled based on a detailed analysis of the results of laboratory and practical tests of cosmetic products based on patent CZ 302 297 B6 [34] specified above, whose detailed formulation was optimized for products, for example with regard to helping the elimination of symptoms of opportunistic infections in the oral cavity *Chytra houba® Pythie® BioPlus,* in the area of non-healing wounds *Biomycosin,* on the surface of the membranes of the urogenital tract *FoelFresh,* and with regard to eliminating the agents of fungal infections of the skin *Chytrá houba® Pythie® Biodeur® Nail* and the veterinary product *Chytrá houba® Ecosin.* All these preparations, containing only *Pythium oligandrum* as an active substance underwent toxicological and safety tests according to the principles of European notification of cosmetic products with optimized formulation, or, in the case of the veterinary preparation, were approved by the Institute for State Control of Veterinary Biologicals and Medicines (Ústav pro státní kontrolu biopreparátů a léčiv Brno), whereby evaluations of the safety of preparations are available from the CPNP European notification portal. Individual preparations formulated in this way were then tested in clinical trials under conditions corresponding to stage II of the clinical trials required for the development of medications,

## Example 1

## (Figure 1A, Figure 1B, Figure 1C, Figure 2)

The preparation *Chytrá houba® Pythie® BioPlus*, in the form of effervescent tablets was tested in a multicentric study incorporating three stomatological clinics, and one clinical microbiology department. Groups of participants in the study suffering from a periodontal disease (periodontitis) were gathered at three dental clinics in Prague and Kladno in the Czech Republic, and in Košice in the Slovak Republic. The group in Prague consisted of 7 participants, the group in Kladno of 15 participants, and the group in Košice of 22 participants. The overall number of participants in the study was, therefore, 44. A control group was treated in parallel using the same method, receiving not a biological, but the classic chemical preparation chlorhexidine gel. The participants in the study were regularly monitored with measurements of PBI (Papilla Bleeding Index), which reflects the immediate condition of the inflammation in the gums, and of CPITN (Community Periodontal Index of Treatment Needs), which is a gauge of the long-term condition of the teeth and a predictor of needs from the perspective of their treatment. The study proceeded using the double-blind method, in which envelopes with the preparations were prepared and numbered by an administration worker not actually involved in the course of the study. After carrying out dental hygiene and signing informed consent, the individual patients were instructed how to conduct their evening dental hygiene and apply the preparations. Application was undertaken in the form of rinsing every evening after completing oral hygiene for five consecutive days. A dental check-up was conducted at the beginning of the study, after 2 months and after 6 months, whereby the condition of the teeth was checked, and, at the end of the study after six months, the colonization of the microbe of the healthy oral microbiome evaluated based on a genetic test.

It was ascertained in a statistical evaluation of the results that the effect of the chemical disinfectant chlorhexidine was only short-term, since there was a statistically significant reduction of the PBI with $p \leq 0.05$ only after 2 months of monitoring, and not at the end of the study 4 months later. By contrast, the long-term results in the group using the biological preparation were excellent. A statistically significant reduction of the PBI ($p \leq 0.05$) was achieved in the short-term evaluation, and a statistically highly significant reduction of PBI ($p \leq 0.01$) was achieved in the long-term evaluation. Further, stabilization of the condition of the teeth after the period of 6 months, for which the participants in the study were monitored, was observed. However, the results were heterogeneous in the largest group of participants at the dental clinic in Košice, and the expected curative effects were not achieved after statistical evaluation, in contrast to the two groups specified above. In order to clarify this situation, details of the clinical records were used, and the complete microbial profile of the oral microflora was monitored in this group of participants using a genetic test before applying the preparation. A mutual correlation of clinical and laboratory results made it possible to state that this heterogeneous group consists of three sub-groups, indicated as low-risk, medium-risk and high-risk groups. The striking feature of the high-risk group is the low content of physiological oral bacteria, the so-called green protective complex bacteria, incorporating the analyzed strains *Eikenella corrodens* and *Capnocytophaga sputigena.*

The results are shown in **Figures 1A, 1B and 1C. Figure 1A** shows the values of the PBI (Papilla Bleeding Index) for, on the one hand, patients indicated in the graph as "unresponsive patients" under conditions corresponding to application of a preparation with *Pythium oligandrum,* and, on the other, patients indicated in the graph as "responsive patients" under conditions corresponding to the application of the dual microbial preparation, all these before application, 2 months after application and 6 months after application.

At the beginning of the study, before application (black column), there was no significant statistical difference between the groups. The "unresponsive patients" had a PBI value of $38.8 \pm 5.8$ and the "responsive patients" had a PBI value of $44.6 \pm 4.7$. The probability value of the dissimilarity of both groups was $p = 0.121$; meaning that both groups of patients were not therefore significantly statistically different. At the end of the study, after 6 months, the "unresponsive patients", under conditions corresponding to the application of *Pythium oligandrum,* showed PBI values that were statistically higher than the "responsive patients", under conditions corresponding to the application of the dual microbial preparation according to the invention. The calculated bleeding index values were after 6 months $39.4 \pm 10.8$ for patients under conditions corresponding to the application of *Pythium oligandrum* alone, whereas the values under conditions corresponding to the application of the dual microbial preparation according to the invention were $10.6 \pm 2.4$. The value of the probability of dissimilarity for both groups was $p = 0.0003$. The results to concern the CPITN (Community Periodontal Index of Treatment Needs) were similar; however, this is a less immediate indicator given its slower reaction to the condition of the oral cavity, and therefore rather reflects long-term trends.

**Figure 1B** shows the dependency of the intensity of hybridization of genetic probes which detect individual groups of oral microorganisms pertaining to the green, orange or red complex. **Figure 1B** shows the results of a genetic analysis of the presence of selected microbes of the green oral complex (light-gray column), the orange oral complex (dark-gray column) and red oral complex (black column) specified in units, corresponding to the number of disintegration per minute of radioactively labeled DNA probes. Participants in the control group, under conditions corresponding to the application of *Chytrá houba® Pythie® BioPlus* (on the right of Figure 1B), and in the experimental group, under conditions corresponding to the application of the dual microbial preparation (on the left of Figure 1B), have a high content of microbes of the orange and red complex, which supports their diagnosis of serious periodontal disease. No statistically significant differences were observed between the two groups. Evident in the experimental group of "responsive patients", however, is the statistically significantly higher content of bacteria of the green complex: $14908 \pm 1489$ *ru* as opposed to $2750 \pm 1171$ *ru*, $p<0.001$, in particular the *Capnocytophaga* bacterium, as is shown in **Figure 1C** (dark column).

These results confirm that the dual microbial preparation according to the invention for use in the oral cavity shows statistically significantly better results in comparison with the standard preparation. Based on these results, we considered and looked for a solution for the effective and long-term effect of the *Pythium oligandrum* oomycete in eliminating the symptoms of periodontitis and gingivitis, and tested and developed experiments to prove the cooperation of this microorganism, *Pythium oligandrum,* with the microbial components of the healthy oral microbiome, in particular with the *Capnocytophaga sputigena* bacterium.

A total of three versions of the dual microbial preparation were prepared for use in the oral cavity and the stability of such preparations was proven, clinical tests also being carried out in patients with periodontal disease. A detailed description of the results obtained is presented hereunder in more detail in this exemplary embodiment of this invention. Altogether, the results of this testing proved that when applying the dual microbial preparation according to the invention in the form of effervescent tablets for oral rinsing, which is the form preferred by most users of the mono-component preparations, there was no negative influence on the stability of any of the components during storage in dry state. After the application of the dual preparation in the form of rinsing of the oral cavity, a simple process for users, there was effective colonization and strengthening of the components of the normal oral microbiome, which led to a significant increase in the success rate of eliminating the symptoms of the person under evaluation according to standard indexes.

*1. The preparation, control and testing of the effectiveness of a dual microbial preparation suitable for eliminating the symptoms of gingivitis and periodontal disease in oral cavity.*

1.1 *Protocol for the preparation of a dual microbial preparation for use in the oral cavity.*

1.1.1 Preparation of the technical preparation Pythium oligandrum M1 ATCC 38472.

The inoculum for the cultivation of the microscopic oomycete *Pythium oligandrum*, strain M1 ATCC 38472, is prepared using the "master cell bank" - "working cell bank" corporate system. The proprietary strain *Pythium oligandrum* M1 ATCC 38472 is stored in the master cell bank by way of long-term freeze drying. A limited quantity of aliquots is produced from the master cell bank, according to the protocol, for the working cell bank so that the number of reproductive generations does not exceed a total of 50. This ensures the genetic stability of the proprietary strain. The cultivation of the inoculum proceeds on a reciprocating shaker with swing of 10.5 cm and 96 swings per a minute for a period of 48 hours at a temperature of 28 °C.

In the meantime, we prepare a millet substrate for the solid cultivation of *Pythium oligandrum* M1: 500 g of hulled *Panicum miliaceum* millet is long-term freezing in the liquid nitrogen during - 150 °C. In 200 ml distilled water are then dissolved: 50 mg $ZnSO_4$, 150 mg $KH_2PO_4$, 50 mg $MgSO_4$ and 250 mg $CaCl_2$. The solution is then heated to boiling point and poured on the washed millet. After boiling, the vessel holding the millet is covered and left to stand for 30 minutes. A thin layer of millet is then spread out on filter paper and left to cool and dry.

Thirty grams of the millet prepared in this was is then added to a 500 ml Erlenmeyer flask, and sterilization proceeds in a steam autoclave at a temperature of 120 °C for a period of 40 minutes. Sterilization is repeated after 24 hours and the millet is shaken slightly after each cycle of sterilization.

We inoculate the sterilized millet with 4 ml of inoculum prepared using the procedure presented above. After inoculation, the millet is shaken thoroughly with the inoculum and finally evened-out into a uniform layer with a light tap.

Solid cultivation on the millet substrate is conducted in a thermal regulator at a temperature of 28 °C and a relative humidity of 70 % for a period of 14 days. The fermented substrate, with *Pythium oligandrum* culture growth, is then dried at 30 °C in a drier for a period of 48 hours until reaching a final humidity of 5 %.

The fermented substrate obtained in this way is ground using a ball mill into particles of less than 0.6 mm in size.

The number of oospores per 1 g of preparation and viability are then determined in the preparation of technical quality prepared in this way according to the procedures presented below. Around 1 g of the prepared technical preparation of *Pythium oligandrum* is weighed precisely on analytical scales and mixed for 1 minute in a mixer in distilled water so that a concentration of exactly 2 g per liter is maintained. Then around 1 ml of the suspension is transferred to a Sedgewick-Rafter counting chamber and the individual characteristic oospores and the oospores in clusters are counted under a microscope so that a minimum of 100 oospores is counted in total. The entire measurement is repeated three times, whereby the suspension of oospores is carefully mixed before each sample is taken to the chamber. Finally, the number of oospores per 1 g of preparation is calculated using the formula

$$n = (a.c)/(b.d.e),$$

where

a is the number of oospores identified;
b is the volume of one square in the chamber (1x1x0.1mm = 1 x $10^{-4}$ ml);

c is the volume of distilled water used to prepare the sample in ml (500 ml);

d is the weight of the sample in g (1 g), and;

e is the number of squares in the chamber in which the identified number of oospores was actually counted.

The germination (viability) of the technical preparation *Pythium oligandrum* is determined by precisely weighing approximately 10 mg of the technical preparation on analytical scales, and mixing it in water with a vortex mixer at a concentration of 1 mg per ml. We prepare three subsequent tenfold dilutions using the suspension prepared in this way, and then plated 100 $\mu$l of each dilution on a MEA (Melt Extract Agar) medium.

We take readings of germination after 8 hours and 16 hours of cultivation in a drier at 28 °C, and then take readings of the final number of colonies after 7 days of cultivation.

The number of oospores obtained ranges from 0.8 to 1.4 x $10^6$ per gram of preparation according to this invention depending on the batch used. Germination (viability) usually ranges from 2 to 10 %. The batch used in example of implementation 1 had 1.0 x $10^6$ oospores per g and germination of 13.1 %, meaning that it contained 0.131 x $10^6$ colony forming units (CFU) per 1 g.

1.1.2 Preparation of the technical preparation *Capnocytophaga sputigena* CCM3712.

The original *Capnocytophaga sputigena* CCM3712 culture is stored at a temperature of -70 °C after delivery from the collection of microorganisms. For cultivation, a small amount of the culture is first transferred to a dish with "chocolate agar", containing trypticase soy agar with 0.1 % yeast extract and 5 % defibrinated horse blood. The dishes were cultivated in 5 % $CO_2$ at 37 °C overnight. The next day, the colonies were transferred to a liquid culture containing trypticase soy substrate comprising 0.1 % yeast extract, 0.002 % equine hemine III, 0.0001 % menadione and 0.1 % sodium bicarbonate. The culture was shaken in an atmosphere of 5 % $CO_2$ in an orbital shaker at 200 revolutions per minute. As soon as the culture reached the middle of the logarithmic phase of $10^9$ cells per ml, the bacteria were sedimented with centrifugation 10000 x $g_{av}$ for a period of 20 minutes and then washed three times in 0.1 M trisodium citrate buffer solution, pH 6.0. After the final wash, the bacteria sediment was carefully separated from the remainder of the buffer solution and dried using freeze drying (lyophyilization).

One gram of lyophilized bacteria containing $10^9$ CFU per one milligram of powder was obtained from one liter of the culture using this procedure.

1.1.3 Preparation of the final formula of the dual preparation for use in the oral cavity.

Three different version of the preparation were used to prepare the final formula, differing from each other in terms of their relative content of *Capnocytophaga sputigena* CCM3712 bacterium. These three versions were marked with the abbreviated names *PlaqueA, PlaqueB and PlaqueC.* The preparations were prepared for pressing into effervescent tablets of a total weight of 3 g for one application and for this reason individual formulae are converted to this weight. The components of individual preparations are shown in Table 1.

The *Pythium oligandrum* component is contained in the dual microbial preparation *PlaqueA, PlaqueB* and *PlaqueC* in a quantity of 0.6663 x $10^4$ CFU per 1 g.

The *Capnocytophaga sputigena* CCM3712 component is contained in dual microbial preparation *PlaqueA* in a quantity of 0.333 x $10^8$ CFU per 1 gram; in the *PlaqueB* preparation in a quantity of 0.333 CFU x $10^9$ per 1 gram; and in the *PlaqueC* preparation in a quantity of 3.333 x $10^{10}$ CFU per 1 gram.

**Table 1**

| **Components of dual microbial preparations for use in the oral cavity** (mg per 3 g tablet)<br>Treatment consists of 5 applications (5 tablets). | | | | |
|---|---|---|---|---|
| Name of preparation | | *PlaqueA* | *PlaqueB* | *PlaqueC* |
| Name of substance | CAS No. | Content (mg) | | |
| *Pythium ol.* | See 1.1.1 | 150 (20x$10^3$ CFU) | 150 (20x$10^3$ CFU) | 150 (20x$10^3$ CFU) |
| *Capnocytoph.* | See 1.1.2 | 0.1 ($10^8$ CFU) | 1 ($10^9$ CFU) | 10 ($10^{10}$ CFU) |
| Sorbitol | 50-70-4 | 719.9 | 719 | 710 |
| PEG 6000 | 25322-68-3 | 90 | 90 | 90 |
| Menth. aroma | - | 30 | 30 | 30 |

(continued)

| Components of dual microbial preparations for use in the oral cavity (mg per 3 g tablet) Treatment consists of 5 applications (5 tablets). | | | | |
|---|---|---|---|---|
| Name of preparation | | *PlaqueA* | *PlaqueB* | *PlaqueC* |
| Name of substance | CAS No. | Content (mg) | | |
| Silicon oxide | 7631-86-9 | 210 | 210 | 210 |
| Citric acid | 77-92-9 | 930 | 930 | 930 |
| NaHCO$_3$ | 144-55-8 | 810 | 810 | 810 |
| Na$_2$CO$_3$ | 497-19-8 | 60 | 60 | 60 |
| Total (mg) | | 3000 | 3000 | 3000 |

1.2 *Test of stability of dual microbial preparation for use in the oral cavity.*

The test of the stability of the dual microbial preparation for use in the oral cavity must ascertain whether both microbial components have acted negatively on each other during the period of storage of the prepared preparation, at the very least for the period of conducting an *in vitro* and *in vivo* effectiveness test. One tablet with the *PlaqueA, PlaqueB* or *PlaqueC* preparation is dissolved in 100 ml of lukewarm water at a temperature of around 35 °C. After it has dissolved completely, 1 ml is taken to determine the germination of *Pythium* and 0.1 ml to determine the CFU of *Capnocytophaga.*

In order to determine CFU in *Capnocytophaga,* we diluted the sample using a series of samples with tenfold serial dilution. In order to determine CFU, we use the third dilution for *PlaqueA,* for example dilution of 10$^3$ x, the fourth dilution of PlaqueB, for example dilution of 10$^4$ x, and the fifth dilution for *PlaqueC,* for example dilution of 10$^5$x.

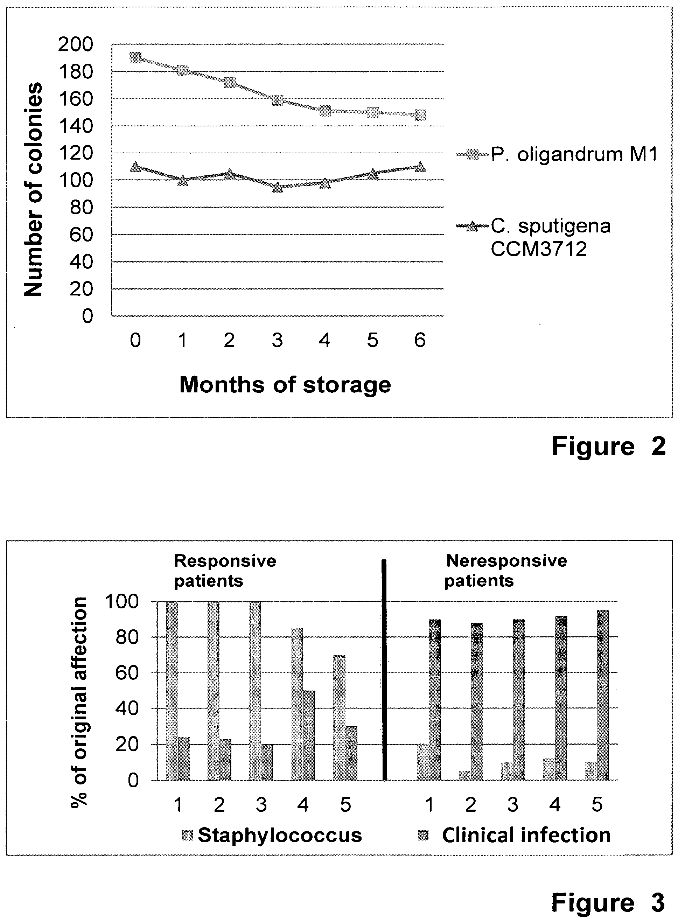

The stability test is conducted immediately after the preparation has been made and again after 1, 2, 3, 4, 5 and 6 months. The results of a typical stability test are shown in **Figure 2,** which depicts the dependency of the number of colonies on the months of storage. It can be concluded from the result that the emergence of *Pythium* during six months of storage and subsequent use of the preparation firstly declined somewhat and then stabilized at a value corresponding to approximately 75 % of the originally-declared nominal value. By contrast, for *Capnocytophaga* there were no significant changes in viability during the test period, viability remaining at the originally declared value. The result shown in **Figure 2** relates to the preparation termed *PlaqueB.* The results obtained with the *PlaqueA* and *PlaqueC* preparations were essentially identical and therefore are not shown in the graph (to ensure that the graph is clearer).

It can be stated, therefore, that neither pressing the original active components into an effervescent tablet nor its activation before use of the dual microbial preparation according to this invention reduced in any significant way the viability of any component of the dual preparation for use in the oral cavity.

1.3 *Effectiveness test of prepared mixtures with the use of an in vitro test.*

The aim of *in vitro* effectiveness tests is to prove that the combination of the *Pythium oligandrum* M1 ATCC38472 oomycete and the bacterium of the green oral complex *Capnocytophaga sputigena* CCM3712 will act synergistically, meaning that there will be an increase in certain measurable *in vitro* activity of the *Pythium oligandrum* oomycete. From this perspective, a standard laboratory test was chosen of suppressing the growth of *Candida albicans* pathogenic yeasts of three different hypha-forming strains obtained from clinical isolates at the hospital in Pardubice, the Czech Republic, and transferred for further experiments to the Institute of Microbiology at the Czech Academy of Sciences (laboratory of Dr. Kolarík). The actual conducting of the test consisted of the use of a standard application record that is invariably commenced by dissolving a tablet with a content of Pythium alone (the *Chytrá houba® Pythie® BioPlus* product) or one of the test tablets specified above, termed *PlaqueA, PlaqueB* and *PlaqueC.* The tablets were dissolved in 100 ml of lukewarm water at a temperature of approximately 35 °C. A quantity of 12.5 ml of this suspension was then mixed directly with 12.5 ml double-concentrate mixture for the preparation of CDA (Czapek Dox Agar) agar plates. 10$^5$ *Candida albicans* pathogenic yeasts were then evenly applied to the plates prepared in this way after cooling. The results of this test are shown below in **Table 2.** The results show that whereas *Pythium oligandrum* alone had a significant influence on suppressing the growth of yeasts in the experiment dishes, its combination with *Capnocytophaga* produced a further strengthening of the effect in the case of all three pathogenic yeasts obtained from clinical isolates. After a week of cultivation at 28 °C, a reading was taken of the number of colonies formed in comparison with the control dish, whereby the reduction in the number of colonies in contrast to the control is a gauge of the effectiveness of *Pythium.* The most effective preparation among the yeast strains under *in vitro* conditions was preparation *PlaqueB,* with the preparations termed *PlaqueA* and *PlaqueC* achieving approximately 80 % of this effectiveness.

With regard to the demanding nature of clinical trials conducted with a large quantity of dual microbial preparation according to this invention, *PlaqueB,* as the most effective preparation, was chosen as the preparation for preclinical and clinical trials.

**Table 2**

| Testing dual preparations for the oral cavity in an *in vitro* test | | | | | | |
|---|---|---|---|---|---|---|
| | C. albicans 2944 | | C. albicans 2548 | | C. albicans 2508 | |
| | Number of colonies in dish | Relative effectiveness against control | Number of colonies in dish | Relative effectiveness against control | Number of colonies in dish | Relative effectiveness against control |
| Control | 954 | - | 1014 | - | 1253 | - |
| Pythium | 330 | 65,4 | 450 | 55,6 | 432 | 65,5 |
| PlaqueA | 285 | 70,1 | 230 | 77,3 | 253 | 79,8 |
| PlaqueB | 95 | 90,0 | 85 | 91,6 | 80 | 93,6 |
| PlaqueC | 273 | 71,4 | 243 | 76,0 | 260 | 79,2 |

**Example 2**

**(Figure 3, Figure 4)**

The cosmetic preparation *Biomycosin,* with active substance *Pythium oligandrum,* was tested on two groups of patients, diabetic and non-diabetic, with microbially inflamed non-healing wounds at the request of the Regional Hospital in Pardubice. The participants in the study were not randomized, but their diabetic status was monitored, which made it possible to divided data into a diabetic and a non-diabetic group at the end of the study. As far as the non-diabetic patients are concerned, their average age was 63.7 $\pm$ 13.1 years, whereby the patients were within an age range of 37 - 79 years. The total number of monitored patients was 19, 14 women and 5 men, because the incidence of inflamed varicose ulcers is higher in women than in men. The application of *Biomycosin* was conducted at the hospital under the supervision of medical staff. Out-patients were hospitalized for the period of application of the preparation. One pack of *Biomycosin* was used for three (eight hours each) wet applications, whereby the applications were made over a total of four consecutive days. Samples were taken three days after the final application for microbiological examination and out-patients were discharged. Regular monthly controls were then conducted in out-patients and hospitalized patients for a period of 6 months following application. The results unambiguously showed a significant reduction in the microbial burden, in particular in G-rods, anaerobic microorganisms and pathogenic yeasts. A negative correlation between a reduction in the microbial burden of G9+ cocci and an improvement in the condition of the clinical infection in non-diabetic patients was clearly proven in a correlative analysis of the results obtained for such patients. This negative correlation was particularly striking for the category of "other staphylococci", of which the *Staphylococcus epidermidis* bacterium in particular is of fundamental importance to the skin. The negative correlation (y) for other staphylococci was characterized by the equation $y = -0.56x + 85.8$, with a correlation coefficient of $R^2 = 0.87$.

With respect to the dependency of the course of treatment in this illness on age and sex, because women are more susceptible, patients first had to be divided into two groups and randomized depending on age and sex. The average age of the patients in the control group corresponding to the conditions of treatment with a preparation containing only P. *oligandrum* was 77.6 $\pm$ 9.8 years. This parameter was 78.0 $\pm$ 10.3 years in the experimental group corresponding to treatment with a dual preparation. Therefore, there was no statistically significant difference between the ages of the patients in both groups. Three women and 2 men were included in both groups and the percentage of women in both groups was therefore identical at 67 %. The results obtained after both methods of application are shown in **Figure 3.** The reciprocal relationship between the quantity of removed staphylococci and the reduced improvement of clinical symptoms of infection is absolutely clear from the results obtained. A high content of maintained staphylococci during treatment with the preparation having a content of the *P. oligandrum* oomycete is absolutely clear for the clinically responsive patients shown in **Figure 3,** on the left-hand side, and a significant improvement in the clinical signs of infection, mostly 20 % to 50 % of the original condition, is also clear. By contrast, the group of clinically unresponsive patients, whose results are shown on the right-hand side of **Figure 3,** show a very distinctive reduction in the number of staphylococci identified by cultivation (including the *Staphylococcus epidermidis* strain); however, progress in removing the clinical symptoms of infection was very low in these patients.

Based on these results, we considered and verified the hypothesis that suppressing certain G+ cocci was not desirable since it affected the physiological microflora representative. We tested this working hypothesis by preparing a total of three versions of the dual microbial preparation according to this invention for use in the long-term elimination of the symptoms of clinical infection in non-healing wounds. The stability of such preparations was proven and their effectiveness was monitored using the relevant laboratory test. A detailed description of the results obtained is presented hereunder in more detail in this example. Altogether, the results of this testing proved that there was no negative influence on any of the components of the dual preparation when storing the form of a dry preparation. In comparison with the one-component preparation used so far, with a *Pythium oligandrum* base, there was a statistically significant reduction in the microbial yeast load and an improvement in the development of clinical infection when using the microbial dual preparation.

*2. The preparation, control and testing of the effectiveness of a dual microbial preparation suitable for eliminating the symptoms of clinical infection in non-healing wounds in non-diabetics.*

*2.1 Protocol for the preparation of a dual microbial preparation for use on non-healing wounds in non-diabetics.*

2.1.1 Preparation of the technical preparation Pythium oligandrum M1 ATCC 38472.

The procedure used for this preparation is identical to the procedure described in Section 1.1.1.

The batch used in exemplary embodiment 2 had $1.1 \times 10^6$ oospores per g and germination of 14.3 %, meaning that it contained $0.157 \times 10^6$ colony forming units (CFU) per 1 g.

2.1.2 Preparation of the technical preparation Staphylococcus epidermidis CCM2124.

The original *Staphylococcus epidermidis* CCM2124 culture is stored at a temperature of -70 °C after delivery from the collection of microorganisms. For cultivation, a small amount of the culture is first transferred to a dish with the tryptone soya agar CM131 <u>Oxoid</u>. The dishes were cultivated at a temperature of 37 °C overnight. Ten well-separated colonies were transferred to a 2 liter Erlenmeyer flask containing 500 ml of medium comprising 30 g per liter peptone, 10 g per liter yeast autolysate, 5 g/l NaCl, 0.1 g $CaCl_2.2H_2O$ and 1 ml trybutyrin; the pH of the medium was regulated at 8.0. The bacteria were cultivated to the middle of the logarithmic phase, sedimented with centrifugation 10000 x $g_{av}$ for a period of 20 minutes and then washed three times in 0.1 M trisodium citrate buffer solution, pH 6.0. After the final wash, the bacteria sediment was carefully separated from the remainder of the buffer solution and dried using freeze drying (lyophilization).

Approximately 0.6 g of lyophilized bacteria containing $10^8$ CFU per one milligram of powder was obtained from one liter of the culture.

2.1.3 Preparation of the final formula of a dual microbial preparation for use on non-healing wounds in non-diabetics.

Three different versions of the preparation were used in order to prepare the final formula, differing in terms of their relative content of the *Staphylococcus epidermidis* CCM2124 bacterium; these three versions were designated using the abbreviated working titles of *NonhealA, NonhealB* and *NonhealC*. The preparations were prepared for use in one package containing 10 g of loose preparation for one application. The components of the individual preparations are presented below in Table 3 on the next page.

The *Pythium oligandrum* component is contained in the dual microbial preparation *NonhealA* and *NonhealB* in a quantity of $7.85 \times 10^3$ CFU per 1 g and in *NonhealC* in a quantity of $7.85 \times 10^3$ CFU per 1 g.

The *Staphylococcus epidermidis* CCM2124 component is contained in the dual microbial preparation *NonhealA in a quantity of* $1 \times 10^6$ CFU per 1 g; in the *NonhealB* preparation *in a quantity of* $1 \times 10^7$ CFU per 1 g; and in the *NonhealC* preparation in a quantity of $1 \times 10^8$ CFU per 1 g.

*2.2 Stability test of dual microbial preparation for use on non-healing wounds in non-diabetics.*

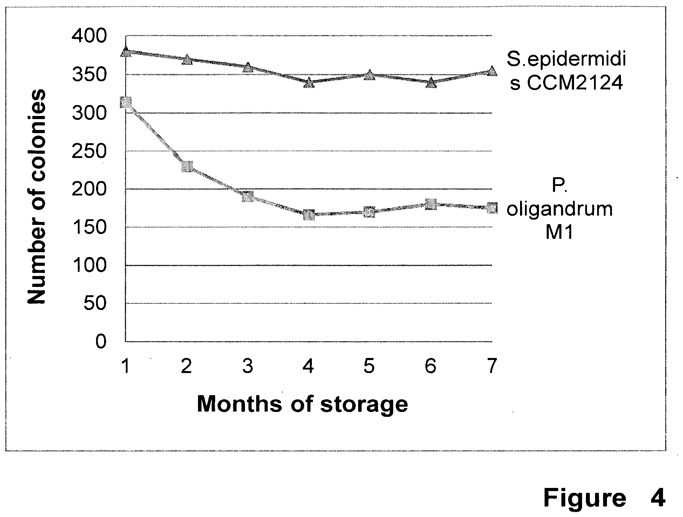

The test of the stability of the dual microbial preparation for use on non-healing wounds must ascertain whether both microbial components have acted negatively on each other during the period of storage of the prepared preparation, at the very least for the period of conducting *in vitro* and *in vivo* effectiveness tests. One package, 10 g of the *NonhealA, NonhealB* or *NonhealC* preparation, is re-suspended in 250 ml of lukewarm physiological solution 9 g/l NaCl at a temperature of around 35 °C. After complete resuspension, 1 ml is taken to determine the germination of Pythium and 0.1 ml to determine CFU of staphylococcus. In order to determine CFU for staphylococcus, we further dilute the sample using tenfold serial dilutions, and to determine CFU we use 1X dilution for *NonhealA,* for example dilution of 10 x, 2X dilution for *NonhealB,* for example dilution of $10^2$ x, and 3X dilution for *NonhealC,* for example dilution of $10^3$ x. The stability test is conducted immediately after the preparation has been made and again after 1, 2, 3, 4, 5 and 6 months. The results of a typical stability test are shown in **Figure 4.** It can be concluded from the result that the viability of Pythium during six months of storage and subsequent use of the preparation firstly declined somewhat and then stabilized at a value corresponding to approximately 75 % of the originally-declared nominal value. By contrast, for *staphylococcus* there were no significant changes in viability during the test period, viability remaining at almost the originally declared value. The result shown in **Figure 4** relates to the preparation termed *NonhealB*. The results obtained with the *NonhealA and NonhealC* preparations were very similar and therefore are not shown in the graph (to ensure that the graph is clearer).

**Table 3**

| Components of dual microbial preparations for use on non-healing wounds in non-diabetics | | | | |
|---|---|---|---|---|
| Components are stated in mg per 10 g of loose preparation. | | | | |
| 1 treatment consists of 4 applications (4 packs of the preparation). | | | | |
| Name of preparation | | *NonhealA* | *NonhealB* | *NonhealC* |
| Name of substance | CAS No. | Content (mg) | | |
| *P. oligandrum* | See 2.1.1 | 500 (78x$10^3$CFU) | 500 (78x$10^3$CFU) | 500 (78x$10^3$CFU) |
| *Staphylococcus.* | See 2.1.2 | 0.1 ($10^7$ CFU) | 1 ($10^8$ CFU) | 10 ($10^9$ CFU) |
| Silicon oxide | 7631-86-9 | 9499.9 | 9499 | 9490 |
| Total (mg) | | 10000 | 10000 | 10000 |

It can therefore be stated that the viability of any of the components of the dual microbial preparation for use on non-healing wounds was not reduced by drying the original active components and storing them in the presence of silica gel without activation before the use of the preparation according to this invention.

*2.3 Effectiveness test of prepared mixtures of dual microbial preparation using an in vitro test.*

The aim of *in vitro* effectiveness tests is to prove that the combination of the Pythium oligandrum M1 ATCC38472 oomycete and the bacterium of the normal skin microbiome *Staphylococcus epidermidis* CCM2124 will act synergicaly, meaning that there will be an increase in certain measurable *in vitro* activity of the Pythium oligandrum oomycete. From this perspective, a standard laboratory test was chosen of suppressing the growth of *Candida albicans* pathogenic yeasts of three different hypha-forming strains obtained from clinical isolates at the hospital in Pardubice, the Czech Republic, and transferred for further experiments to the Institute of Microbiology at the Czech Academy of Sciences. The actual conducting of the test consisted of the use of a standard application protocol, which was invariably commenced by re-suspending a dry preparation containing only Pythium (the *Biomycosin* preparation) or one of the above-mentioned test preparations designated as *Nonheal A, B* or *C*. Preparations were re-suspended in 250 ml of lukewarm physiological solution at a temperature of around 35 °C. A quantity of 12.5 ml of this suspension was then mixed directly with 12.5 ml double-concentrate mixture for the preparation of CDA (Czapek Dox Agar) agar plates. $10^5$ *Candida albicans* pathogenic yeasts obtained from clinical isolates was then evenly applied to the plates prepared in this way after cooling. After a week of cultivation at 28 °C, a reading was taken of the number of colonies in comparison with the control dish, whereby the reduction in the number of colonies in contrast to the control is a gauge of the effectiveness of *Pythium*. The results of this test are shown below in Table 4. The results show that whereas *Pythium* alone had a significant influence on suppressing the growth of yeasts in the experiment dishes, its combination with staphylococcus produced a further strengthening of the effect. The most effective preparation among the three pathogenic yeast strains under *in vitro* conditions was preparation *NonhealB,* with the preparations termed *NonhealA* and *NonhealC* achieving approximately 50 % of this effectiveness.

With regard to the demanding nature of clinical trials conducted with a large quantity of dual microbial preparation according to this invention, the most effective preparation, NonhealB, was proposed as the starting preparation for preclinical and clinical trials.

**Table 4**

| Testing dual microbial preparations for non-healing wounds in an *in vitro* test | | | | | | |
|---|---|---|---|---|---|---|
| Strain number | *C. albicans 2944* | | *C. albicans 2548* | | *C. albicans 2508* | |
| Monitored Parameter | Number of colonies in dish | Relative effectiveness against control | Number of colonies in dish | Relative effectiveness against control | Number of colonies in dish | Relative effectiveness against control |
| Control | 980 | - | 1013 | - | 988 | - |
| *Pythium* | 335 | 65,8 | 364 | 64,1 | 370 | 62,6 |
| *NonhealA* | 270 | 72,4 | 255 | 74.8 | 262 | 73,5 |
| *NonhealB* | 102 | 89,6 | 98 | 90,3 | 107 | 89,2 |
| *NonhealC* | 283 | 71,1 | 248 | 75,5 | 260 | 73,7 |

The example shown is not limited to the use of the dual microbial preparation specified in this exemplary embodiment for non-healing wounds on skin. There are naturally other possible uses not specified here; for example use in different cases of opportunistic microbial infections accompanied by dysbiosis on the skin, such as atopic eczema and psoriasis.

**Example 3**

**(Figure 5A, Figure 5B, Figure 5C, Figure 6)**

Convincing evidence was taken from clinical observations in the area of care for oral cavity health, non-healing wounds and suppressing yeasts in vaginal candidiasis that *Pythium oligandrum* suppresses and kills pathogenic yeasts of the *Candida* and *Malassezia* strains. This conclusion was then independently confirmed by a laboratory cultivation test at the Institute of Microbiology of the Czech Academy of Sciences in Prague. A total of 4 clinical strains of the pathogenic yeast *Candida albicans,* isolated at Pardubice Hospital, were tested in a laboratory competition test, in that this ascertained their ability to pass to hyphal growth, which acts as evidence of the clinical aggression of these strains. The strains involved were *Candida albicans* 2508, 2548, 2558 and 2944. *Pythium oligandrum* fully outgrew all four tested strains in the competition test in the case of using MEA (malt extract agar) as the medium and PDA (potato dextrose agar) as the medium. After 10 days of the experiment, meaning once the experiment had come to an end, the remains of the yeast strains overgrown with Pythium were transferred to a medium of CDA (Czapek-Dox agar). Yeasts grow in this medium, but not *Pythium oligandrum.* Nonetheless, there was no growth of yeasts in this test medium, which shows that *Pythium oligandrum* not only overgrew the pathogenic yeast cells, but killed them. Therefore, following on from the results of these laboratory test results, systematic studies were conducted in cooperation with doctors at gynecological surgeries in Prague, Rychnov nad Kněžnou, Vysoké Mýto, Klášterec nad Ohří, Pilsen and Uhlířské Janovice comparing the effects of the chemical antimycotic *Clotrimazol* and the *FeelFresh* preparation among women with recurring incidence of vaginal candidiasis. A clinical evaluation of the women was conducted by a doctor at the beginning of testing on a six-point scale that considered discomfort, the presence of vaginal discharge, irritation, pain, burning and reddening. An initial microbiological examination was conducted to identify the presence of pathogenic yeasts of the *Candida* strain and of lactobacilli, which are the dominant microorganisms in the normal vaginal microbiome. Application was made for a period of 5 consecutive days, either *Clotrimazol* being applied or a hip bath with the *FeelFresh* preparation being used. A microbiological control was then conducted after 10 days and 1 month. A final clinical study and a microbiological control were then carried out three months following application. The intensity of clinical symptoms in the control group with the *Clotrimazol* application and in the experimental groups with the *FeelFresh* application was comparable at the beginning of the study. After 3 months, however, the results of assessing clinical symptoms (the number of clinical symptoms identified) were statistically better for the group applying *FeelFresh* in comparison with the chemical antimycotic *Clotrimazol*: 2.57 $\pm$ 0.53 and 1.00 $\pm$ 1.52, p=0.035. It was possible to monitor the intensity of yeast infection by way of cultivation after 10 days, 1 month and 3 months, meaning more frequently than the clinical picture. The results were also very interesting in this regard since they showed a rapid and statistically significant reduction in the incidence of pathogenic yeasts for *Clotrimazol* after 10 days, when the initial values in arbitrary units were 3.71 $\pm$ 0.75 and 3.71 $\pm$ 0.48 for the application with *Clotrimazol* and *FeelFresh.* After 10 days, however, the corresponding values in arbitrary units were 1.28 $\pm$ 0.48 and 1.85 $\pm$ 0.38, p=0.04. Nonetheless, after 1 month, or even 3 months, the situation had reversed completely and the reduced level of pathogenic yeasts was stabilized only among patients using *FeelFresh,* when the values after 1 month for control and application in arbitrary units of *FeelFresh* were 2.42 $\pm$ 0.53 and 1.28 $\pm$ 0.48, p=0.008. The values in arbitrary units after 3 months were 3.14 $\pm$ 0.69 and 1.42 $\pm$ 0.78, p=0.007. The positive correlation between improvement in clinical condition and the concentration of lactobacilli on the one hand and between the elimination of yeast infection and the concentration of lactobacilli on the other was also very interesting. These results clearly show, as with the previous examples presented, that the curative effect of the *Pythium oligandrum* microorganism contained in the *FeelFresh* preparation is significantly potentiated by the presence of healthy microflora, in this case lactobacilli. A working hypothesis could be drawn from these results that the presence of lactobacilli significantly increases the effectiveness of suppressing yeasts through the *Pythium oligandrum* oomycete.

A study to prove the role of lactobacilli proceeded using the double-blind method, in which envelopes with the preparations were prepared and numbered by an administration worker not actually involved in the course of the study. After confirming the clinical diagnosis in the first microbiology sample, the patients signed informed consent and were instructed how to apply the preparations. The application was made in the form of a rinse over five consecutive days using a provided vaginal applicator. A gynecologist undertook a professional examination at the beginning of the study and again after 3 months, in that the level of yeast infection and the level of colonization of the vaginal membrane with lactobacilli were invariably checked. The results of the clinical study are clearly presented in graphic format in **Figure 5A, 5B and 5C.**

From the perspective of clinical evaluation at the beginning and at the end of the study, the control group, applying the classic preparation containing only *Pythium oligandrum,* is variable: there was effective elimination of symptoms among certain women, but not in other patients, as is clear from **Figure 5A.** For this reason patients were divided into two groups of clinically responsive patients on the left-hand side of **Figure 5** and clinically unresponsive patients on the right-hand side of the figure.

There was a significant reduction in the incidence of the *Candida albicans* yeast among clinically responsive patients after three months in all cases, whereas there was no such reduction among unresponsive patients (one patient actually experienced an increase - **Figure 5B**). Nonetheless, it is still interesting that the incidence of lactobacilli was far higher among responsive patients in all cases, increasing to physiologically high values during the study (**Figure 5C**), whereas the incidence of yeast was considerably lower among unresponsive patients, accompanied in two cases by further reduction.

These results confirm that the dual microbial preparation for use in suppressing and eliminating pathogenic yeasts shows statistically significantly better results in comparison with a standard preparation in all parameters.

We therefore verified and tested this hypothesis: a total of three versions of the dual microbial preparation according to this invention were prepared for use for vaginal candidiasis, cavity and the stability of such preparations was proven, clinical tests also being carried out in patients with such a diagnosis. A detailed description of the results obtained is presented in Example 3 for this invention below. The aggregate results of this testing showed that the effective microbiological components had no negative influence on each other during drying and storage under dry conditions. The results of laboratory tests show the greatest effectiveness in the dual preparation VaginalB.

*3. Preparation, control and testing of the effectiveness of the dual microbial preparation according to this invention for application on skin and membrane which is susceptible to the incidence of pathogenic yeasts.*

*3.1 Protocol for the preparation of the dual microbial preparation for application on skin and membrane which is susceptible to the incidence of yeasts.*

3.1.1 Preparation of the technical preparation *Pythium oligandrum* M1 ATCC 38472.

The procedure used for this preparation is identical to the procedure described in Section 1.1.1. The batch used in exemplary embodiment 3 had 0.8 x $10^6$ oospores per g and germination of 12.8 %, meaning that it contained 0.102 x $10^6$ colony forming units (CFU) per 1 g of preparation.

3.1.2 Preparation of the technical preparation *Lactobacillus crispatus* CCM7010.

The original *Lactobacillus crispatus* CCM7010 culture is stored at a temperature of -70 °C after delivery from the collection of microorganisms. For cultivation, a small amount of the culture is first transferred to an agar dish with medium for the cultivation of lactobacilli, containing 5 g per liter yeast autolysate, 10 g per liter bovine extract, 10 g per liter peptone, 20 g per liter glucose, 5 ml per liter Tween 80, 2 g per liter $K_2HPO_4$, 5 g per liter sodium acetate, 2 g per liter diamonium citrate, 0.2 g per liter $MgSO_4.7H_2O$ and 0.05 g per liter $MnSO_4.7H_2O$. We regulate the pH after adding all components at a value of pH 6.2 - 6.6. The dishes were cultivated at a temperature of 37 °C overnight. Ten well-separated colonies were transferred to a 2-liter Erlenmeyer flask containing 500 ml of medium 6, having the components specified above. The bacteria were cultivated to the middle of the logarithmic phase, sedimented with centrifugation

10000 x $g_{av}$ for a period of 20 minutes and then washed three times in 0.1 M trisodium citrate buffer solution, pH 6.0. After the final wash, the bacteria sediment was carefully separated from the remainder of the buffer solution and dried using freeze drying (lyophilization).

Approximately 0.8 g of lyophilized bacteria containing $10^9$ CFU per one mg of powder was obtained from one liter of the culture.

3.1.3 Preparation of the final formula of the dual microbial preparation for application on skin and membrane which is susceptible to the incidence of yeasts.

Three different versions of the preparation were used in order to prepare the final formulation, differing in terms of their relative content of the *Lactobacillus crispatus* CCM7010 bacterium; these three versions were designated using the abbreviated titles of *VaginalA*, *VaginalB* and *VaginalC.* The preparations were prepared for use in one package containing 2 g of loose preparation for one application. The components of the individual preparations are presented below in **Table 5** on the next page. The active components in the preparation are *Pythium* and *Lactobacillus.* Silica gel is added as a drying agent in order to preserve the original properties and the emergence of both active components. A small amount of added sodium chloride aids better activation of the biological agent, chamomile aroma acts as perfume.

The *Pythium oligandrum* component is contained in the dual microbial preparation *VaginalA* and *Vaginal B* in a quantity of 19.2 x $10^3$ CFU per 1 g and in *VaginalC* in a quantity of 19.2 x $10^3$ CFU per 1 g.

The *Lactobacillus crispatus* CCM7010 component is contained in the dual microbial preparation VaginalA in a quantity of 0.5 x $10^8$ CFU, in the *VaginalB* preparation in a quantity of 0.5 x $10^9$ CFU per 1 g and in the *VaginalC* preparation in a quantity of 0.5 x $10^{10}$ CFU per 1 g.

*3.2 Stability test of dual preparation for use on skin and membrane which is susceptible to the incidence of yeasts.*

The test of the stability of the dual microbial preparation for use on skin and membrane which is susceptible to the incidence of yeasts must ascertain whether both microbial components have acted negatively on each other during the period of storage of the prepared preparation, at the very least for the period of conducting *in vitro* and *in vivo* effectiveness tests. One pack of 2 g of the *VaginalA*, *VaginalB* or *VaginalC* preparation is re-suspended in 500 ml of lukewarm water at a temperature of around 35 °C. After complete resuspension, 1 ml is taken to determine the germination of *Pythium oligandrum* and 0.1 ml to determine the CFU of lactobacillus units. In order to determine CFU for lactobacillus, we further dilute the sample using tenfold serial dilutions, and to determine CFU we use 1X dilution for *VaginalA* - dilution of 100 x - 2X dilution for *VaginalB* - dilution of $10^3$ x - and 3X dilution for *VaginalC* - dilution of $10^4$ x. The stability test is conducted immediately after the preparation has been mixed and again after 1, 2, 3, 4, 5 and 6 months. The results of a typical stability test are shown in Figure 6. It can be concluded from the result that the viability of *Pythium* during six months of storage and subsequent use of the preparation firstly declined somewhat and then stabilized at a value corresponding to approximately 80 % of the originally-declared nominal value. By contrast, there was only a slight reduction in viability of around 10 % for lactobacillus during the test period. The result shown in Figure 6 relates to the preparation termed *VaginalB.* The results obtained with the *VaginalA* and *VaginalC* preparations were very similar and therefore are not shown in the graph (to ensure that the graph is clearer).

**Table 5**

| Components of dual preparations for use on skin and membrane which is susceptible to the incidence of yeasts  Components are stated in mg per 2 g of loose preparation.  1 treatment consists of 5 applications (5 preparations). | | | | |
|---|---|---|---|---|
| Name of preparation | | *VaginalA* | *VaginalB* | *VaginalC* |
| Name of substance | CAS No. | Content (mg) | | |
| *Pythium oligandrum* | See 3.1.1 | 376 (38.4 x $10^3$ CFU) | 376 (38.4 x $10^3$ CFU) | 376 (38.4 x $10^3$ CFU) |
| *Lactobacillus* | See 3.1.2 | 0.1 ($10^8$ CFU) | 1 ($10^9$ CFU) | 10 ($10^{10}$ CFU) |
| Silicon oxide | 7631-86-9 | 1618.9 | 1618 | 1609 |
| NaCl | 7647-14-5 | 4 | 4 | 4 |
| Chamomile oil | 8022-66-2 | 1 | 1 | 1 |
| Total (mg) | | 2000 | 2000 | 2000 |

It can therefore be stated that the emergence of any of the components of the dual microbial preparation for use in the oral cavity was not significantly reduced by drying the original active components and storing them in the presence of silica gel or its activation before the use of the preparation according to this invention.

*3.3. Effectiveness test of prepared mixtures with the use of an in vitro test.*

The aim of *in vitro* effectiveness tests is to prove that the combination of the *Pythium oligandrum* M1 ATCC38472 and healthy bacterium of the normal vaginal microbiome *Lactobacillus crispatus* CCM7010 will act synergicaly, meaning that there will be an increase in certain measurable *in vitro* activity of the *Pythium oligandrum* oomycete. From this perspective, a standard laboratory test was chosen of suppressing the growth of *Candida albicans* pathogenic yeasts of three different hypha-forming strains obtained from clinical isolates at the hospital in Pardubice, the Czech Republic, and transferred for further experiments to the Institute of Microbiology at the Czech Academy of Sciences (laboratory of Dr. Kolarik). The actual conducting of the test consisted of the use of a standard application protocol, which was invariably commenced by re-suspending a dry preparation containing only *Pythium oligandrum* (the *FeelFresh* preparation) or one of the above-mentioned test preparations designated as *VaginalA*, *VaginalB* or *VaginalC*. Preparations were re-suspended in 500 ml of lukewarm water of a temperature of around 35 °C and 12.5 ml of this suspension was then mixed directly with 12.5 ml of double-concentrate mixture, cooled to a temperature of 45 °C, for the preparation of CDA (Czapek Dox agar) agar plates. $10^5$ *Candida albicans* pathogenic yeasts obtained from clinical isolates were then evenly applied to the plates prepared in this way after cooling. After a week of cultivation at 28 °C, a reading was taken of the number of grown colonies in comparison with the control dish, whereby the reduction in the number of colonies in contrast to the control is a gauge of the effectiveness of *Pythium oligandrum.* The results of this test are shown below in **Table 6.** The results show that whereas *Pythium oligandrum* alone had a significant influence on suppressing the growth of yeasts in the experiment dishes, its combination with lactobacillus produced a further strengthening of the effect. The most effective preparation among the three pathogenic yeast strains under *in vitro* conditions was the *VaginalB* preparation, with the preparations termed *VaginalA* and *VaginalC* achieving approximately 50 % of this effectiveness.

With regard to the demanding nature of clinical trials conducted with a large quantity of preparation, *VaginalB,* as the most effective dual microbial preparation, was chosen for preclinical and clinical trials.

**Example 4**

**(Figure 7)**

The significant positive correlation between the presence of yeasts and improvement in the clinical condition of infection in non-healing wounds was seen in the same way for diabetic and non-diabetic patients. The relevant correlation coefficients were 0.4 and 1.0.

**Table 6**

| Testing dual microbial preparations for suppressing yeasts in an *in vitro* test | | | | | | |
|---|---|---|---|---|---|---|
| | *C. albicans 2944* | | *C. albicans 2548* | | *C. albicans 2508* | |
| | Number of colonies in dish | Relative effectiveness against control | Number of colonies in dish | Relevant effectiveness against control | Number of colonies in dish | Relevant effectiveness against control |
| Control | 1013 | - | 998 | - | 1120 | - |
| *Pythium* | 302 | 70.2 | 325 | 67.4 | 332 | 70.3 |
| *VaginalA* | 272 | 73.1 | 265 | 73.4 | 273 | 75.6 |
| *VaginalB* | 102 | 89.9 | 103 | 89.5 | 89 | 86.7 |
| *VaginalC* | 273 | 73.1 | 273 | 72.6 | 267 | 76.2 |

This correlation led to consideration of whether the presence of yeasts or their components might cause activation of the *Pythium oligandrum* microorganism. With respect to the fact that biological tests concurrently containing three different microorganisms are very complicated from the perspective of their execution and evaluation, alternatives were chosen including dead *Saccharomyces cerevisiae* baker's yeasts in the form of so-called yeast autolysate, which is commonly commercially available. A standard plate test on an agar MEA was used. Its components were as follows:

20 g per liter malt extract, 20 g per liter glucose, 1 g per liter peptone and 20 g per liter agar. Interaction was monitored of the *Pythium oligandrum* oomycete and the dermatophytes *Trichophyton interdigitale* DMF2477 and *Microsporum fulvum* P245, for which previously tests were conducted to show that they suppress the growth of these dermatophytes to around 50 % uninhibited growth. However, *Pythium* was activated after adding yeast extract in a concentrate of 1 g per liter to the agar carrier and there was an increase in growth inhibition to 75 % for Trichophyton and 80 % for Microsporum. Therefore, this result also showed how the result of the biological abilities of *Pythium* to suppress certain dermatophytes is dependent on its physiological state, in that its biological ability could be significantly enhanced by the presence of even inactivated target organisms or another form of their processed components and extracts, in this case commercial yeast autolysate. A working hypothesis could be drawn from these results that even inactivated yeasts have an influence on increasing the effectiveness of the *Pythium oligandrum* oomycete.

The optimum dual preparation to eliminate the agents of mycosis (dermaphytosis) was tested in patients with mycotic diseases of the feet incorporating dermatophytic infection on the soles, between the toes and under the nails - onychomycosis. The curative effect was clinically evaluated by dermatologists and microbiological tests were also carried out at the beginning and the end of the period under consideration. Patients were randomized into two groups. In the first group, treatment was provided with a one-component preparation with *Pythium oligandrum* oomycete content and in the second group a combined preparation with yeast autolysate content was administered. The results showed good clinical effectiveness for the preparation containing only *Pythium oligandrum*, but the clinical effectiveness for the dual microbial preparation was even better (control 3.0 $\pm$ 0, experiment 3.6 $\pm$ 0.5, t-test, p=0.01). The improvement in microbiology according to Figure 9C was similar (control 2.0 $\pm$ 0, experiment 2.6 $\pm$ 0.5, t-test, p=0.01).

These results confirm that the dual microbial preparation according to the invention for use on mycosis of the feet shows statistically significantly better results in comparison with the standard one-component preparation.

A total of 25 Cavia *porcellus* guinea pigs underwent a study to monitor the effects of a one-component preparation containing the *Pythium oligandrum* oomycete. The effect of the preparation was evaluated by veterinarians and confirmed by taking microbiological samples at the beginning and at the end of the period under consideration, whereby all specimens were negative for the presence of dermatophytic funguses and yeasts at the end of the experiment. The overall score of the clinical evaluation of the effects of the preparation was 1.72 $\pm$ 0.87, p < 0.05, in that 1 = excellent effect and 4 = no effect. The zoonotic characteristic of dermatophytic infections which scientists frequently describe guarantees us that the mechanism of these infections in animals is identical to mycotic infections in humans. With respect to the demanding nature of clinical tests and the fact that the results obtained in evaluating the effectiveness of the one-component preparation corresponded to them in a comparison of tested human and animal groups, section 4.3 may be referred to for further verification of the effectiveness of the dual preparation.

Both series of the experiments carried out above showed the potentiating influence of yeasts or products of their metabolism on the effectiveness of preparations containing *Pythium oligandrum* in eliminating the agents of dermatophytosis. This potentiating effect could be caused by the activation by live yeasts or the activation of their metabolites without the immediate requirement of the incidence of live yeasts. The final option showed itself to be more likely in systematic research and the hypothesis was therefore put forward that *Pythium oligandrum* is stimulated in its abilities to eliminate dermatophytes by certain components relating to the yeast metabolism.

This working hypothesis was therefore tested: a total of three versions of the dual microbial preparation according to this invention were prepared for use on mycosis of the feet and the stability of such preparations was proven, clinical tests also being carried out on patients having this disease. A detailed description of the results obtained is presented in Example 4 for this invention. Altogether, the results of this testing showed that, in comparison with the live version of dual microbial preparations, a relatively large quantity of the inactivated, dead partner microorganism or its components must be added, the addition of up to around 10 weight percent proving effective in the case of the yeast autolysate tested. There really was a statistically significant increase in effectiveness in the preparation prepared in this way against the classic one-component preparation. This increase was equivalent to the increase observed in the case that the yeast infection appeared endogenously as a result of naturally-occurring co-infection, which occurs in approximately 20 % of cases.

*4. The preparation, control and testing of the effectiveness of the dual microbial preparation according to this invention as suitable for application in the case of mycosis of the feet, including onychomycosis in people and dermatophytes in animals.*

*4.1 Protocol for the preparation of a dual preparation for use on mycosis in humans and animals.*

4.1.1 Preparation of the technical preparation *Pythium oligandrum* M1 ATCC 38472. The procedure used for this preparation is identical to the procedure described in Section 1.1.1. The batch used in exemplary embodiment 4 had 1.3 x $10^6$ oospores per g and germination of 14.5 %, meaning that it contained 0.189 x $10^6$ colony forming units (CFU) per 1 g of preparation.

4.1.2 Yeast autolysate, the second component in the preparation, was bought from Oxoid.

4.1.3 Preparation of the final formula of the dual preparation for application on mycosis.

Three different version of the preparation were used to prepare the final formula, differing from each other in terms of their relative content of yeast autolysate. These three versions were given the abbreviated names of *MycosinA, MycosinB* and *MycosinC.* The preparations were prepared for pressing into effervescent tablets of a total weight of 3 g and for this reason individual formulae are converted to this weight. The components of individual preparations are shown below in **Table 7.**

The *Pythium oligandrum* component is contained in the dual microbial preparation *MycosinA, MycosinB* and *MycosinC* in a quantity of $12.6 \times 10^3$ CFU per 1 g.

The content of *yeast autolysate* is contained in the *MycosinA* dual microbial preparation in a quantity of 50 mg per 3 g tablet, meaning a quantity of 1.66 % weight for one tablet; in the *MycosinB* preparation in a quantity of 100 mg per 3 g tablet, meaning a quantity of 3.33 % for one tablet; and in the *MycosinC* preparation in a quantity of 150 mg per 3 g tablet, meaning a 5 % weight for one 3 g tablet.

**Table 7**

| Components of dual preparation for use on mycosis and dermatophytosis Components are stated in mg for one 3 g tablet. 1 treatment consists of 3 applications (3 tablets). | | | | |
|---|---|---|---|---|
| *Name of preparation* | | *MycosinA* | *MycosinB* | *MycosinC* |
| Name of substance | CAS No. | Content (mg) | | |
| *Pythium oligandrum* | See 4.1.1 | 200 ($37.8 \times 10^3$ CFU) | 200 ($37.8 \times 10^3$ CFU) | 200 ($37.8 \times 10^3$ CFU) |
| Yeast autolysate | See 4.1.2 | 50 | 100 | 150 |
| Silicon oxide | 7631-86-9 | 110 | 60 | 10 |
| Sorbitol | 50-70-4 | 1260 | 1260 | 1260 |
| Citric acid | 77-92-9 | 690 | 690 | 690 |
| NaHCO3 | 144-55-8 | 540 | 540 | 540 |
| PEG 6000 | 25322-68-3 | 90 | 90 | 90 |
| Na2CO3 | 497-19-8 | 60 | 60 | 60 |
| Total (mg) | | 3000 | 3000 | 3000 |

*4.2 Stability test of the dual preparation suitable for application in the case of mycosis and dermaphytosis.*

A stability test was not conducted for the preparation in question since it is well-known from literary sources that the presence of yeast autolysate in a dried preparation does not influence the properties of the *Pythium oligandrum* oomycete.

*4.3 Effectiveness test of prepared mixtures with the use of an in vitro test.*

The aim of *in vitro* effectiveness tests is to prove that the combination of *Pythium oligandrum* M1 ATCC38472 oomycete and inactivated yeast components contained in yeast autolysate acts synergicaly, meaning that there is an increase in certain measurable *in vitro* activity of the *Pythium oligandrum* oomycete. From this perspective, a standard laboratory test was chosen of suppressing the growth of four types of dermatophytes, for which it is proven that they are known agents of mycotic diseases of a zoonotic character. Specifically, the tests were conducted with the use of four common dermatophytes, two different strains of each being used: *Trichophyton interdigitale* (Ti), *Trichophyton erinacei* (Te), *Microsporum fulvum* (Mf) and *Microsporum canis (Mc).* The results of determining the activities of individual preparations are presented in **Figure 7**, in that the number of formed colonies of dermatophytes is related to the control without an active substance. The results shown in **Figure 7** concern the *MycosinB* preparation, the results obtained with the *MycosinA* and *MycosinC* preparations being qualitatively similar, but with resultant ability to suppress dermatophytes of only around 30 % of the increase on the control. The result in **Figure 7** clearly shows that a simple preparation containing only *Pythium oligandrum* as an active substance has the ability to suppress dermatophytes, suppression at a level of 50 - 60 % of the control growth being achieved. Yeast autolysate itself had no ability to suppress the growth of dermatophytes in the test used, the results being at roughly the same level as the control experiment. A statistically significant increase in effectiveness was shown for the dual preparation at a level of approximately 10 % of infections in control samples.

With regard to the demanding nature of clinical trials conducted with a large quantity of preparations according to this invention, the most effective preparation, MycosinB, was proposed as the starting preparation for preclinical and clinical trials.

**Example 5**

The activation of the other unique abilities of the microscopic oomycete *Pythium oligandrum* by the components of healthy microflora is also worth noting. The unique ability of our technical substance, *Pythium oligandrum,* to disrupt the biofilms formed by pathogenic bacteria populating non-healing wounds was proven in our previous studies. The results of the study were presented at the 12th international "Interdisciplinary collaboration in the treatment of wounds and skin defects" congress held on 23 and 24 January at the Faculty of Health Studies at the University of Pardubice. The measurements conducted showed that 129 of the 160 tested bacterial strains clinically isolated from non-healing wounds created biofilm. A 70 % reduction in the creation of biofilm was also proven in biofilm-positive strains after the addition of germs of the *Pythium oligandrum* oomycete using the *Biomycosin* preparation, in that the reduction of biofilm was very significant in 43 % of the tested strains, there occurring a reduction in the activity of biofilm creation of more than 50 %. No change in the intensity of creation of biofilm was recorded in 17 % of the strains examined *in vitro,* while no reproducible results were obtained in the remaining 13 % of the monitored strains. It was possible to influence the production of biofilms at least *in vitro* in the case of the observed *Stenotrophomonas maltophilia* and *Pseudomonas aeruginosa* strains. If, however, bacteria of the normal skin microbiome *Staphylococcus epidermidis* were added to the incubation mixture in addition to germs of the oomycete, there was a reduction in the creation of biofilms of more than 50 % even in the two awkward strains already mentioned. This interesting phenomenon, together with the knowledge already published [32], shows that in addition to their metabolic regulatory functions, the components of the normal physiological microbiome might also play a part in reducing the intensity of biofilm created with the participation of pathogenic microorganisms.

The aim of *in vitro* effectiveness tests is to prove that the combination of the *Pythium oligandrum* M1 ATCC38472 oomycete and the healthy bacterium of the normal skin microbiome will act synergicaly, meaning that there will be an increase in certain measurable *in vitro* activity of the *Pythium oligandrum* oomycete. Another extremely important factor which must be verified is the specificity of such action. Components of the physiological microbiome were often applied in the previous experiments in probiotic preparations, without of course maintaining topological specificity. The effectiveness of preparations provided in this way was mostly very low, which correlates well with the differing microbial composition of physiological microbiomes in topologically different locations of the human body with different levels of exposure to the outside environment and different metabolic niches. A simple laboratory test of the creation of biofilms was therefore used to verify this hypothesis, as is described in this exemplary embodiment. The creation of biofilm was measured based on the absorption of blue microbial dye, depicting the intensity of the creation of biofilm, whereby each experiment was conducted three times independently in triplicate [38]. This viability was measured using a test of vital staining according to literature [39] in order to prove the influence of the viability of the microorganisms in biofilms.

The results of these determinations, conducted with preparations containing only the microscopic oomycete *P. oligandrum* according to invention CZ 302 297 B on the one hand and with the optimized dual microbial preparations described in exemplary embodiments 1, 2 and 3 according to the invention submitted on the other, unambiguously showed that the optimum disruption of biofilms always occurred only in the case of dual preparations comprising the physiological microbial component contained in the microbiome of the relevant topology. Biofilms of the oral cavity, therefore, were most effectively disrupted by a dual microbial preparation containing the dominant physiological microbe for this location, whereas the effect of other physiological components was minor. Similar evidence of specificity was also shown in the case of the skin and vaginal microbiome.

The results obtained in this way are very important because they show that there is no universal probiotic or dual microbial preparation which is suitable for long-term suppression of the symptoms of opportunistic microbial infections - only a preparation having a content of topologically relevant microbial components is invariably effective.

**Table 8**

| Influence of dual microbial preparations according to patent CZ 302 297 B and according to this invention on the creation and activity of microbial communities **creating a biofilm in the oral cavity** (specific monitoring of the creation of biofilm for the *Streptococcus gordonii* and *Fusobacterium nucleatum* bacteria). | | |
|---|---|---|
| *Preparation used* | *Creation of biofilm ($A_{590}$)\** | *Activity of biofilm ($A_{490}$)\** |
| Control | $1.05 \pm 0.15$ | $0.90 \pm 0.04$ |
| Chytrá houba® Pythie BioPlus | $0.69 \pm 0.10$ | $0.57 \pm 0.04$ |
| Plaque B | $0.12 \pm 0.08$ | $0.10 \pm 0.01$ |
| Biomycosin | $0.70 \pm 0.11$ | $0.56 \pm 0.03$ |
| Nonheal B | $0.72 \pm 0.10$ | $0.53 \pm 0.04$ |
| Feel Fresh | $0.50 \pm 0.08$ | $0.45 \pm 0.03$ |
| Vaginal B | $0.51 \pm 0.07$ | $0.44 \pm 0.04$ |
| \*The average value of absorbance $\pm$ standard deviation calculated from three independent determinations, each of which was conducted in triplicate (three independent measurements). | | |

*5.1 The influence of dual microbial preparations on the disruption of biofilms containing oral cavity bacteria.*

In this test, simple laboratory tests were conducted on the creation and viability of biofilms using two oral cavity bacteria, *Streptococcus gordonii* and *Fusobacterium nucleatum*, as the monitored components. Both these bacteria have a key role in the creation of microbial biofilms of the oral cavity: *Streptococcus* is the only bacterium capable of directly catching on tooth enamel in the aggressive environment of the oral cavity, whereas the large (in terms of dimensions) bacterium of the *Fusobacterium* genus creates important retaining centers for the colonization of bacteria of the orange and red complex in invasive pathogens of type *Aggregatibacter actinomycetemcommitans.* The results of the laboratory test are shown in **Table 8** (above). The results clearly show that whereas the influence on biofilm was lesser for a simple preparation containing only the P. *oligandrum* oomycete, only the use of the *PlaqueB* dual preparation led to very marked disruption and restriction of the viability of the oral biofilm. The somewhat more significant disruption of biofilm caused by the FeelFresh preparation and *VaginalB* can be ascribed to the higher percentage content of Pythium in this preparation.

*5.2 The influence of dual microbial preparations on the disruption of biofilms containing bacteria in non-healing wounds*

In this test, simple laboratory tests were conducted on the creation and viability of biofilms using two bacteria creating biofilms in non-healing wounds, *Stenotrophomonas maltophilia* and *Pseudomonas aeruginosa*, as the monitored components These bacteria were used for the reason that in previous tests conducted by Dr. Karel Mencl at the Department of Clinical Microbiology at Pardubice Hospital, these two strains were resistant to the disruption of biofilm using simple preparations having only the *Pythium oligandrum* oomycete. The results shown in **Table 9** obviously clearly show that a dual microbial preparation intended for improving clinical infection in non-healing wounds and containing the normal skin microbiome component *Staphylococcus epidermidis* was able to effectively overcome this shortcoming and ensure effective disruption of biofilm in these resistant types. A certain lesser influence was also recorded for the *VaginalB* preparation containing lactobacilli, for which such activity has been described [33].

**Table 9**

| Influence of dual microbial preparations according to patent CZ 302 297 B2 and according to this invention on the creation and activity of microbial communities **creating biofilm in non-healing wounds** (specific monitoring of the creation of biofilm for the *Stenotrophomonas maltophilia* and *Pseudomonas aeruginosa* bacteria). | | |
|---|---|---|
| *Preparation used* | *Creation of biofilm ($A_{590}$)\** | *Activity of biofilm ($A_{490}$)\** |
| Control | $1.10 \pm 0.25$ | $0.86 \pm 0.05$ |
| Chytrá houba® Pythie BioPlus | $0.70 \pm 0.08$ | $0.58 \pm 0.03$ |
| Plaque B | $0.68 \pm 0.07$ | $0.56 \pm 0.04$ |

(continued)

| Influence of dual microbial preparations according to patent CZ 302 297 B2 and according to this invention on the creation and activity of microbial communities **creating biofilm in non-healing wounds** (specific monitoring of the creation of biofilm for the *Stenotrophomonas maltophilia* and *Pseudomonas aeruginosa* bacteria). | | |
|---|---|---|
| Preparation used | Creation of biofilm $(A_{590})$* | Activity of biofilm $(A_{490})$* |
| Biomycosin | 0.60 ± 0.10 | 0.50 ± 0.03 |
| Nonheal B | 0.13 ± 0.07 | 0.10 ± 0.01 |
| Feel Fresh | 0.50 ± 0.08 | 0.44 ± 0.04 |
| Vaginal B | 0.31 ± 0.07 | 0.25 ± 0.02 |
| *The average value of absorbance ± standard deviation calculated from three independent determinations, each of which was conducted in triplicate (three independent measurements). | | |

5.3 *The influence of dual microbial preparations on the disruption of biofilms created by pathogenic yeasts.*

In this test, simple laboratory tests were conducted on the creation and viability of biofilms using the *Candida albicans* yeast as the monitored component. More effective disruption of this biofilm occurred with the use of the dual microbial preparation *VaginalB*, as is evident from the results shown in **Table 10** above. This knowledge could be of general significance, because the pathogenic yeast *Candida albicans* frequently creates combined.

**Table 10**

| Influence of dual microbial preparations according to patent CZ 302 297 B2 and according to this invention on the creation and activity of microbial communities **creating biofilm in areas susceptible to yeast infections** (specific monitoring of the creation of biofilm for the pathogenic yeast *Candida albicans*). | | |
|---|---|---|
| Preparation used | Creation of biofilm $(A_{590})$* | Activity of biofilm $(A_{490})$* |
| Control | 1.08 ± 0.20 | 0.96 ± 0.05 |
| Chytrá Houba Pythie BioPlus | 0.72 ± 0.09 | 0.56 ± 0.04 |
| Plaque B | 0.70 ± 0.08 | 0.55 ± 0.05 |
| Biomycosin | 0.70 ± 0.10 | 0.54 ± 0.02 |
| Nonheal B | 0.68 ± 0.07 | 0.53 ± 0.02 |
| Feel Fresh | 0.50 ± 0.07 | 0.44 ± 0.01 |
| Vaginal B | 0.07 ± 0.02 | 0.06 ± 0.01 |
| *The average value of absorbance ± standard deviation calculated from three independent determinations, each of which was conducted in triplicate (three independent measurements). | | |

5.3. *The influence of dual microbial preparations on the disruption of biofilms created by pathogenic yeasts.*

In this experiment were used the simple laboratory tests and viability of the biofilm using as monitored component *Candida albicans.* The most effective ruption of the biofilm occurred by using the dual microbial preparation *VaginalB,* as is evident from the results in Table 10 the above. These knowledges may have a general significance, because the pathogenic yeast *Candida albicans* often creates the combined microbial biofilms in combination with certain types of pathogenic bacteria.

**Example 6** - **(Figure 8A, Figure 8B)**

This example represents non-claimed comparative embodiment, when is serves the purpose of clarifying the invention.
A major asset of the dual microbial preparations according to the submitted invention is the opportunity to use them for prevention, for which values of active components which are up to ten times lower can be used based on the possibility of effective colonization and long-term propagation at the point of application. The effective mycoparasitism of the *P. oligandrum* oomycete and the anti-fungal action of certain other microbial strains may obviously also be used to protect the living and working environment from molds. It is well described in medical literature that the molds occurring in the living and working environment can shoot harmful spores into their surroundings. If their average concentration in the air exceeds a value of 500 viable spores per 1 m$^3$, harmful effects leading to allergies, respiratory illnesses and even depression could be manifested in full according to the standards set by the World Health Organization (WHO).

The anti-mold product BioRepel, containing *P. oligandrum* and used mainly to eliminatedisb, ceilings, floors and other areas of contaminated rooms, was developed and is successfully used based on invention CZ 302 297. Other microbial preparations that are capable of eliminating mold based on the principle of antibiosis and that primarily use for such purposes the abundantly widespread *Bacillus amyloliquefaciens* bacillus is also used for this purpose.

The effectiveness of a dual preparation in eliminating molds and yeasts from the living environment based on a combination of the two above-mentioned microorganisms is tested in field experiments under the conditions of their actual use. The experimental object is, for example, a wall in a damp room with even incidence of the black mold *Aspergillus niger,* in that we measure the concentration of spores in the rooms before application and 6 months after application and quantitatively evaluate the presence of mold directly in smears of material taken from walls in a standard way. For application, 3 g of the preparation is divided into two bags, bag A containing 1 g of the preparation and bag B containing 2 g of the preparation. Bag A is re-suspended in 10 liters of lukewarm water and the whole of the affected area is rubbed with a sponge soaked in this preparation. After a gentle drying, the same area is rubbed with a sponge soaked in solution B, which is prepared by re-suspending bag B in 1.5 liters of lukewarm water, Samples to ascertain effectiveness are taken after 1 month, 3 months and 6 months, at which time the field experiment is ended. A preparation is considered effective in the case in which the number of spores in the fall is reduced to less than 500 (WHO standard) and the presence of mold ascertained using a cultivation test is reduced to level 1 (present sporadically only after cultivation). The specific result of field experiments at two different locations is depicted in **Figure 8**. It is clear that the required reduction in the concentration of spores in the atmosphere was reduced to a value of less than 500 in both locations observed only when using the dual preparation, even though both its components showed certain reduction against the control (**Figure 8A**). Similarly, only the dual preparation reduced the actual incidence of mold on the wall, proven by a smear test, to the target value of around 1.0. In this case too, the effect of individual preparations was only partial and the target values were not achieved (**Figure 8B**). These results confirm the hypothesis that the dual microbial preparation for the elimination of mold and yeasts from the living environment shows better results than a standard preparation.

We further tested and verified this hypothesis. As part of this verification, we prepared, according to the procedures described in this exemplary embodiment, three different versions of the combined preparation and verified their effectiveness on the target mold under *in vitro* conditions.

*6. The preparation, control and testing of the effectiveness of a dual microbial preparation suitable for the elimination of mold and yeasts from the living environment.*

*6.1 Protocol for the preparation of a dual microbial preparation for use in the elimination of mold and yeasts from the living environment*

6.1.1. Preparation of the technical preparation *Pythium oligandrum* M1 ATCC 38472.

The procedure used for this preparation is identical to the procedure described in Section 1.1.1. The batch used in exemplary embodiment 6 had 1.0 x 10$^6$ oospores per g and germination of 12.6 %, meaning that it contained 0.126 x 10$^6$ colony forming units (CFU) per 1 g of preparation.

6.1.2. Preparation of the technical preparation *Bacillus amyloliquefaciens* CCM1084.

The original *Bacillus amyloliquefaciens* CCM1084 culture is stored at a temperature of -70 °C after delivery from the collection of microorganisms. For cultivation, a small amount of the culture is first transferred to a dish with agar and medium 10 for the cultivation of bacilli comprising peptone, 5 g per liter, bovine extract, 3 g per liter, and $MnSO_4.H_2O$, 0.01 g per liter; the pH of the medium was regulated at 7.0. The dishes were cultivated at a temperature of 37 °C overnight. Ten well-separated colonies were transferred to a 2-liter Erlenmeyer flask containing 500 ml of medium 10, having the components specified above. The bacteria were cultivated to the middle of the logarithmic phase, sedimented with centrifugation 10000 x $g_{av}$ for a period of 20 minutes and then washed three times in 0.1 M trisodium citrate buffer solution, pH 6.0. After the final wash, the bacteria sediment was carefully separated from the remainder of the buffer solution and dried using freeze drying (lyophilization).

Approximately 0.6 g of lyophilized bacteria containing 10$^8$ CFU per one gram of powder was obtained from one liter of the culture.

6.1.3. Preparation of the final formula for a dual preparation for use in eliminating molds and yeasts from the living environment

Three different versions of the preparation were used in order to prepare the final formula, differing in terms of their relative content of the *Bacillus amyloliquefaciens* CCM1084 bacterium; these three versions were designated using the abbreviated titles of MoldA, MoldB, and MoldC. The preparations were prepared for use in one pack containing 3 g of loose preparation (bag A containing 1 g and bag B containing 2 g of loose preparation). The components of individual preparations are shown in **Table 11** below.

**Table 11**

| Components of dual preparations for the elimination of mold and yeasts from the living environment, Components are stated in mg per 3 g of loose preparation. | | | | |
|---|---|---|---|---|
| *Name of preparation* | | *MoldA* | *MoldB* | *MoldC* |
| Name of substance | CAS No. | Content (mg) | | |
| Pythium | See 1.1.1 | 750 ($94.5 \times 10^3$ CFU) | 750 ($94.5 \times 10^3$ CFU) | 750 ($94.5 \times 10^4$ CFU) |
| Bacillus | See 1.1.2 | 0.1 ($10^7$ CFU) | 1 ($10^8$ CFU) | 10 ($10^9$ CFU) |
| Silica gel | 7631-86-9 | 2499,9 | 2499 | 2490 |
| *Total* | | *3000* | *3000* | *3000* |

*6.2 Stability test of dual preparation for the elimination of mold and yeasts from the living environment.*

The test of the stability of the dual preparation for use in eliminating mold and yeasts from the living environment must ascertain whether both microbial components have acted negatively on each other during the period of storage of the prepared preparation, at the very least for the period of conducting *in vitro* and *in vivo* effectiveness tests. One pack (3 g) of the preparation MoldA, MoldB or MoldC is re-suspended in 10000 ml of lukewarm water (temperature of around 35 °C). After complete resuspension, 10 ml is taken to determine the germination of Pythium and 1 ml to determine the CFU of the bacillus. In order to determine the CFU in the bacillus, we further dilute the sample using tenfold serial dilutions, and to determine the CFU we use undiluted preparation for MoldA (dilution of 1 x), 1X dilution for MoldB (diluted of 10 x) and 2X dilution for MoldC (dilution of $10^2$ x). The stability test is conducted immediately after the preparation has been made and again after 1, 2, 3, 4, 5 and 6 months. It can be concluded from the result that the viability of Pythium during six months of storage and subsequent use of the preparation first declined somewhat and then stabilized at a value corresponding to approximately 75 % of the originally-declared nominal value. By contrast, for bacillus there were no significant changes in the viability during the test period, viability remaining at almost the originally declared value.
It can therefore be concluded that the emergence of any of the components of the dual preparation is not significantly affected by drying the original active components and storing them in the presence of silica gel.

*6.3 Effectiveness test of prepared mixtures with the use of an in vitro test.*

The aim of in vitro effectiveness tests is to prove that the combination of the *Pythium oligandrum* M1 ATCC38472 oomycete and the bacterium of the environment *Bacillus amyloliquefaciens* will act synergicaly, meaning that there will be an increase in certain measurable in vitro activity of the *Pythium oligandrum* oomycete. From this perspective, a standard laboratory test was chosen of suppressing the growth of contaminating *Aspergillus niger* mold on walls. The actual conducting of the test consisted of the use of a standard application protocol, which is invariably commenced by re-suspending a dry preparation containing only Pythium (the BioRepel preparation) or one of the above-mentioned test preparations designated as MoldA, B or C. Preparations were re-suspended in 250 ml of physiological saline solution of a temperature of around 35 °C and 12.5 ml of this suspension was then mixed directly with 12.5 ml of double-concentrate mixture for the preparation of MEA agar plates. $10^5$ fungal microconidia *Aspergillus niger* was then applied evenly to the plates prepared in this way after cooling. After a week of cultivation at 28 °C, a reading was taken of the number of colonies formed in comparison with the control dish, whereby the reduction in the number of colonies in contrast to the control is a gauge of the effectiveness of Pythium. The results of this test are shown below in **Table 12**. The results show that whereas Pythium alone had a significant influence on suppressing the growth of yeasts in the experiment dishes, its combination with staphylococcus produced a further strengthening of the effect. The most effective preparation among the three pathogenic yeast strains under *in vitro* conditions was the MoldB preparation, with the preparations termed MoldA and MoldC achieving approximately 50 % of this effectiveness. With respect to the demanding nature of field tests, the most effective preparation, MoldB, is proposed for further field tests.

**Table 12**

| Testing dual preparations for the elimination of mold and yeasts from the environment | | | | | | |
|---|---|---|---|---|---|---|
| | *Aspergillus niger* | | *Aspergillus niger* | | *Aspergillus niger* | |
| | *Number* | *Relative effectiveness* | *Number* | *Relative effectiveness* | *Number* | *Relative effectiveness* |
| Control | 1054 | - | 1064 | - | 1110 | - |
| Pythium | 340 | 67,7 | 350 | 67,1 | 352 | 68,2 |
| MoldA | 287 | 72,8 | 280 | 73,7 | 273 | 75,4 |
| MoldB | 105 | 90,0 | 85 | 92,0 | 80 | 92,8 |
| MoldC | 289 | 72,5 | 283 | 73,4 | 270 | 75,7 |

The example shown, for the use of the dual microbial preparation specified in this exemplary embodiment, is not limited to the elimination of mold or yeasts from the surrounding environment. This type of dual microbial preparation can be positively used as prevention of the incidence of mold and yeasts in the living and working environment, which could be important to people suffering from opportunistic microbial infections.

**Industrial applicability**

The solution is intended for application to ensure a healthy oral cavity, on non-healing wounds such as varicose ulcers, on the skin and to suppress yeasts occurring on the above-mentioned places and on the mucous membrane of the urogenital tract and for other places on the human body, in particular on skin affected by fungal or yeast infections.

**Cited literature**

[1] Aas JA, Paster BJ, Stokes LN et al (2005) J Clin Microbiol 43, 5721-5732.
[2] Ainamo J, Barmes D, Baegric D (1982) Int Dent J 32, 281-289.
[3] Arabatzis M et al (2007) Br J Dermatol 157, 681-689.
[4] Berezov AB, Darveau RP (2011) Periodontal 2000 55, 36-47.
[5] Burne RA, Zeng L, Ahn SJ et al (2012) Adv Dent Res 24, 77-80.
[6] Calderone RA, Fonzi WA (2001) Trends Microbiol 9, 327-335.
[7] US 5,190,746 A (issued March 2, 1993), Cassels JF, London J.
[8] Cutting KF, White R (2004) Br J Community Nurs 9:S6-S15.
[9] Darveau RP (2010) Nat Rev Microbiol 8, 481-490.
[10] DeBernardis F, Liu H, O'Mahony R etal (2007) J Infect Dis 195, 149-157.
[11] Dewhirst FE, Chen T, Izard J et al (2010) J Bacteriol 192, 5002-5017.
[12] EWMA Patient Outcome Group (2010) J Wound Care 19, 239-268.
[13] Findley K, Oh J, Yang J et al (2013) Nature 498, 367-370.
[14] Frandin C, Kretschmar M, Nichterlein T et al (2003) Mol Microbiol 47, 1523-1543.
[15] Gottrup E, Apelqvist J, Bjansholt T et al (2013) J Wound Care 22, S1-S89.
[16] Gow NAR, Hube B (2012) Curr Opin Microbiol 15, 406-412.
[18] Grice EA, Segre JA (2012) Annu Rev Genomics Human Genet 13, 151-170.
[19] Hajishengalis G, Liang S, Payne MA et al (2011) Cell Host & Microbe 10, 497-506.
[20] Holt SC, Ebersole JL (2005) Periodontal 2000 38, 72-122.
[21] Hube B (2004) Curr Opin Microbiol 7, 336-341.
[22] Human Microbiome Project Consortium (2012) Nature 486, 207-214.
[23] Kostov G, Angelev M, Denkova Z et al (2011) Eng Life Sci 11, 517-527.
[24] Kotnik T (2007) Slov Vet Res, 44 (3): 63-73.
[25] Lang NP, Adler R, Joss A et al (1990) J Clin Periodontol 17, 714-721.
[26] WO 2013/122 931 A2 (publ. 22.8.2013), Lanzalaco AC, Charbonneau DL,Howard BW.
[27] Liu B, Faller RR, Klitgord N et al (2012) PLoS One 7, e37919.

[28]Morrell J, Stratman E (2011) J Agromedicine, 16:4, 244-250.

[29]Nakagawa Y, Ohno N, Murai T (2003) J Infect Dis 187, 710-713.

[30]Okuda K, Kato T, Ishihara K (2004) Oral Dis 10, 5-12.

[31] CA 2374938 A1 (publ. 30. 11. 2000) Reid G, Bruce A.

[32] WO2008/077251(publ.3.7.2008), Smoragiewicz W,Karska-Wysocki,Bazo,Ruiz Liquet.

[33]Stapleton AE et al (2011) Clin Infect Dis 52, 1212-1217.

[34] CZ 302 297 B6 (publ. 29.12.2010), Suchánek M, Klimeš R.

[35]Teughels W, Durukan A, Ozcelik O et al (2013) J Clin Periodontol 40, 1025-1035.

[36] CZ 9883 U (publ. 14.4.2000). Veselý, D, Veselý L.

[37]Wounds UK (2010) Aberdeen: Wounds UK 2010. Best practice statement: use of topical antiseptic / antimicrobial agents in wound management.

[38] Moskowitz SM, Foster JM, Emerson J et al (2004) J Clin Microbiol 42, 1915-1920.

[39] Bruzual I, Riggler P, Hadley S et al (2007) J Antimicrob Chemother 59, 441-450.

**Claims**

1. The dual microbial preparation for use in medical treatment of human or animal symptoms of opportunistic microbial infections, in particular symptoms caused by dysbiosis, preferably in the oral cavity, in the skin, and in the vagina, said preparation comprises two basic microbial components in mutual synergic action, i.e. the microscopic oomycete *Pythium oligandrum* and a microbiome component, whereby the microbiome component is a component of a physiological microbiome of human or animal origin.

2. The dual microbial preparation for use according to claim 1 **characterized by the fact** that the microscopic oomycete *Pythium oligandrum* and the physiological microbiome component facilitate their germination, subsequent propagation and colonization at the target tissues.

3. The dual microbial preparation for use according to claims 1 and 2 **characterized by the fact** that the microscopic oomycete *Pythium oligandrum* is incorporated in a quantity of $10^3$ to $10^7$ CFU (colony forming units) per 1 g.

4. The dual microbial preparation for use according to claims 1 to 3 **characterized by the fact** that the microscopic oomycete *Pythium oligandrum* is incorporated in a quantity of $10^4$ to $10^5$ CFU (colony forming units) per 1 g.

5. The dual microbial preparation for use according to claims 1 to 4 **characterized by the fact** that the physiological microbiome component is incorporated in a quantity of $5 \times 10^6$ to $5 \times 10^{10}$ CFU (colony forming units) per 1 g.

6. The dual microbial preparation for use according to claims 1 to 5 **characterized by the fact** that the physiological microbiome component is incorporated in a quantity of $5 \times 10^7$ to $5 \times 10^9$ CFU (colony forming units) per 1 g.

7. The dual microbial preparation for use according to claims 1 to 6 **characterized by the fact** that the fermented substrate found in the *Pythium oligandrum* oomycete is the source of nutrients for both microbial components.

8. The dual microbial preparation for use according to claims 1 to **characterized by the fact** that it also contains at least one auxiliary component from a group comprising:

    a desiccant such as silicon dioxide;
    components of a buffer system such as citric acid, sodium bicarbonate and sodium carbonate;
    an anti-caking substance, such as sorbitol or polyethylene glycol; and,
    an agent for the creation of a physiological osmotic environment, such as sodium chloride.

9. The dual microbial preparation for use according to claims 1 to 8 **characterized by the fact** that the human microbiome component, i.e. one of the components of the healthy oral cavity microbiome, such as the *Capnocytophaga sputigena* bacterium, is the physiological microbiome component.

10. The dual microbial preparation for use according to claims 1 to 8 **characterized by the fact** that the human microbiome component, i.e. one of the components of the healthy skin microbiome, such as the *Staphylococcus epidermidis* bacterium, is the physiological microbiome component,

**11.** The dual microbial preparation for use according to claims 1 to 8 **characterized by the fact** that the human microbiome component, i.e. one of the components of the healthy vaginal microbiome, such as the peroxide-producing *Lactobacillus crispatus,* is the physiological microbiome component.

**12.** The dual microbial preparation for use according to claim 1 **characterized by the fact** that either the microscopic oomycete *Pythium oligandrum* or the component of a physiological microbiome of human or animal origin is present in the form of non-viable components, such as the killed cells, cell extracts or isolated cellular fractions.

**13. The** dual microbial preparation for use according to claim 12 **characterized by the fact** that the non-viable components increasing the effectiveness of *Pythium oligandrum* oomycete is a yeast autolysate in a quantity of 0.1 % to 10 % weight of the total quantity of dual preparation.

**14.** The dual microbial preparation for use according to claim 8 **characterized by** the fact that the auxiliary components allow for application in the form of an ointment, cream, oil or suppository or in the form of a liquid aqueous preparation.

**Patentansprüche**

**1.** Das duale mikrobielle Präparat für die Verwendung bei der Heilung menschlicher oder veterinärer Symptome opportunistischer mikrobieller Infektionen, insbesondere Symptome, die durch Dysbiose vorwiegend in der Mundhöhle, auf der Haut und in der Vagina hervorgerufen werden, umfaßt zwei mikrobielle Grundkomponenten mit gegenseitiger synergetischer Wirkungsweise, und zwar den mikroskopischen Oomyceten *Pythium oligandrum* und eine Komponente des Mikrobioms, wobei die Komponente des Mikrobioms eine Komponente des physiologischen Mikrobioms menschlicher oder tierischer Herkunft ist.

**2.** Das duale mikrobielle Präparat für die Verwendung laut Anspruch 1 **wird dadurch gekennzeichnet, daß** der mikroskopische Oomycete *Pythium oligandrum* und die Komponente des physiologischen Mikrobioms für die Keimung, der sich anschließenden Vermehrung und die Kolonisierung der Zielgewebe behandelt wurden.

**3.** Das duale mikrobielle Präparat für die Verwendung laut der Ansprüche 1 und 2 **wird dadurch gekennzeichnet, daß** der mikroskopische Oomycete *Pythium oligandrum* in einer Menge von $10^3$ bis zu $10^7$ CFU (colony forming units) pro 1 g enthalten ist.

**4.** Das duale mikrobielle Präparat für die Verwendung laut der Ansprüche 1 bis 3 **wird dadurch gekennzeichnet, daß** der mikroskopische Oomycete *Pythium oligandrum* in einer Menge von $10^4$ bis $10^5$ CFU (colony forming units) pro 1 g enthalten ist.

**5.** Das duale mikrobielle Präparat für die Verwendung laut der Ansprüche 1 bis 4 **wird dadurch gekennzeichnet, daß** die Komponente des physiologischen Mikrobioms in einer Menge von $5 \times 10^6$ bis zu $5 \times 10^{10}$ CFU (colony forming units) pro 1 g enthalten ist.

**6.** Das duale mikrobielle Präparat für die Verwendung laut der Ansprüche 1 bis 5 **wird dadurch gekennzeichnet, daß** die Komponente des physiologischen Mikrobioms in einer Menge von $5 \times 10^7$ bis $5 \times 10^9$ CFU (colony forming units) pro 1 g enthalten ist.

**7.** Das duale mikrobielle Präparat für die Verwendung laut der Ansprüche 1 bis 6 **wird dadurch gekennzeichnet, daß** für beide mikrobiellen Komponenten die Nährstoffquelle das im Oomycete *Pythium oligandrum* enthaltene fermentierte Substrat ist.

**8.** Das duale mikrobielle Präparat für die Verwendung laut der Ansprüche 1 bis 7 **wird dadurch gekennzeichnet, daß** es im weiteren mindestens eine der Hilfssubstanzen aus der Gruppe :

die ein Trockenmittel, wie es Siliziumdioxid ist;
Komponenten des Puffersystems wie Zitronensäure, Natriumhydrogencarbonat und Natriumcarbonat;
einen Antiverklumpungsstoff, wie Sorbitol oder Polyäthylenglykol; sowie
ein Mittel zur Bildung einer physiologisch-osmotischer Umgebung, wie es Natriumchlorid ist, enthält.

**9.** Das duale mikrobielle Präparat für die Verwendung laut der Ansprüche 1 bis 8 **wird dadurch gekennzeichnet,**

**daß** die Komponente des physiologischen Mikrobioms eine Komponente des menschlichen Mikrobioms ist, und zwar eine der Komponenten des gesunden Mikrobioms der Mundhöhle, wie es das Bacterium *Capnocytophaga sputigena* ist.

10. Das duale mikrobielle Präparat für die Verwendung laut der Ansprüche 1 bis 8 **wird dadurch gekennzeichnet, daß** die Komponente des physiologischen Mikrobioms eine Komponente des menschlichen Mikrobioms ist, und zwar eine der Komponenten des gesunden Hautmikrobioms, wie es das Bacterium *Staphyloccocus epidermidis* ist.

11. Das duale mikrobielle Präparat für die Verwendung laut der Ansprüche 1 bis 8 **wird dadurch gekennzeichnet, daß** die Komponente des physiologischen Mikrobioms eine Komponente des menschlichen Mikrobioms ist, und zwar eine der Komponenten des gesunden Vaginalmikrobioms, wie es das Peroxid produzierende Bacterium *Lactobacillus crispatus* ist.

12. Das duale mikrobielle Präparat für die Verwendung laut Anspruch 1 **wird dadurch gekennzeichnet, daß** entweder der mikroskopische Oomycete *Pythium oligandrum* oder eine Komponente des physiologischen Mikrobioms menschlichen oder tierischen Ursprungs in Form nichtlebender Bestandteile als abgetötete Zellen, Zellextrakte oder als isolierte Zellfraktionen anwesend sind.

13. Das duale mikrobielle Präparat für die Verwendung laut Anspruch 12 **wird dadurch gekennzeichnet, daß** ein nichtlebendiger Bestandteil, der die Wirkung des Oomycetes *Pythium oligandrum* erhöht, ein Hefeautolysat in einer Menge von 0.1 % bis zu 10 Gew. % von der Gesamtmenge des dualen Präparats ist.

14. Das duale mikrobielle Präparat für die Verwendung laut Anspruch 8 **wird dadurch gekennzeichnet, daß** die Hilfsstoffe die Form von Salben, Cremes, Ölen, Zäpfchen oder die Form eines flüssigen wässrigen Präparats haben.

**Revendications**

1. Double préparation microbienne pour utilisation dans le traitement des symptômes de l'homme ou vétérinaire des infections microbiennes opportunistes, avant tout des symptômes dus à une dysbiose, en priorité dans la cavité buccale, sur la peau et dans le vagin, cette préparation incluant deux composants microbiens de base dans une synergie commune, et cela microscopique l'oomycète *Pythium oligandrum* et un composant du microbiome, le composant du microbiome étant un composant du microbiome physiologique d'origine humaine ou animale.

2. Double préparation microbienne pour utilisation selon la revendication 1 **se caractérisant par le fait que** l'oomycète microscopique *Pythium oligandrum* et le composant du microbiome physiologique sont traités, pour leur germination, multiplication ultérieure et colonisation des tissus cibles.

3. Double préparation microbienne pour utilisation selon les revendications 1 et 2 **se caractérisant par le fait que** l'oomycète microscopique *Pythium oligandrum* est présent dans une quantité de $10^3$ à $10^7$ UFC (unité formant colonie) par gramme.

4. Double préparation microbienne pour utilisation selon les revendications 1 à 3 **se caractérisant par le fait que** l'oomycète microscopique *Pythium oligandrum* est présent dans une quantité de $10^4$ à $10^5$ UFC (unité formant colonie) par gramme.

5. Double préparation microbienne pour utilisation selon les revendications 1 à 4 **se caractérisant par le fait que** le composant du microbiome physiologique est présent dans une quantité de $5 \times 10^6$ à $5 \times 10^{10}$ UFC (unité formant colonie) par gramme.

6. Double préparation microbienne pour utilisation selon les revendications 1 à 5 **se caractérisant par le fait que** le composant du microbiome physiologique est présent dans une quantité de $5 \times 10^7$ à $5 \times 10^9$ UFC (unité formant colonie) par gramme.

7. Double préparation microbienne pour utilisation selon les revendications 1 à 6 **se caractérisant par le fait que** le substrat fermenté contenu dans l'oomycètee *Pythium oligandrum* est une source de nutriments pour les deux composants microbiens.

8. Double préparation microbienne pour utilisation selon les revendications 1 à 7 **se caractérisant par le fait qu'**elle comporte en outre au moins un de composants auxiliaires d'un groupe incluant :

   un dessicant, tel que le dioxyde de silicium ;
   des composants de système tampon, tels que l'acide citrique, le bicarbonate de sodium et le carbonate de sodium ;
   un anti-agglomérant, tels que le sorbitol ou le polyéthylène glycol ; et
   un produit pour la formation d'un milieu physiologique osmotique, tel que le chlorure de sodium.

9. Double préparation microbienne pour utilisation selon les revendications 1 à 8 **se caractérisant par le fait que** le composant du microbiome physiologique est un composant du microbiome humain, et ce un des composants du microbiome sain de la cavité buccale, tel que la bactérie *Capnocytophaga sputigena.*

10. Double préparation microbienne pour utilisation selon les revendications 1 à 8 **se caractérisant par le fait que** le composant du microbiome physiologique est un composant du microbiome humain, et ce un des composants du microbiome sain de la peau, tel que la bactérie *Staphyloccocus epidermidis.*

11. Double préparation microbienne pour utilisation selon les revendications 1 à 8 **se caractérisant par le fait que** le composant du microbiome physiologique est un composant du microbiome humain, et ce un des composants du microbiome sain du vagin, tel que *Lactobacillus crispatus* fabriquant des peroxydes.

12. Double préparation microbienne pour utilisation selon la revendication 1 **se caractérisant par le fait que** l'oomycète microscopique *Pythium oligandrum* ou le composant du microbiome physiologique d'origine humaine ou animale sont présents sous forme de composés inanimés, tels que les cellules mortes, les extraits cellulaires ou les fractions cellulaires isolées.

13. Double préparation microbienne pour utilisation selon la revendication 12 **se caractérisant par le fait qu'**un autolysat de levure dans une quantité de 0,1% à 10 % massiques sur la quantité totale de la préparation microbienne constitue les composés inanimés augmentant l'effet de l'oomycète *Pythium oligandrum.*

14. Double préparation microbienne pour utilisation selon la revendication 8 **se caractérisant par le fait que** de composants auxiliaires existent sous une forme de pommades, de crèmes, d'huiles, de suppositoires ou sous forme de préparation aqueuse.

**Figure 1A**

**Figure 1B**

**Figure 1C**

**Figure 2**

**Figure 3**

Figure 4

Figure 5A

Figure 5B

Figure 5C

Figure 6

Figure 7

**Figure 8A**

**Figure 8B**

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- CZ 9883 U
- CZ 302297
- US 5190746 A
- CA 2374938 A1
- WO 2008077251 A
- WO 2013122931 A2
- US 5190734 A
- CZ 302297 B6
- CZ 302297 B
- CZ 302297 B2

## Non-patent literature cited in the description

- *CHEMICAL ABSTRACTS,* 25322-68-3, 7631-86-9, 77-92-9, 144-55-8, 497-19-8
- *CHEMICAL ABSTRACTS,* 7631-86-9
- *CHEMICAL ABSTRACTS,* 7631-86-9, 7647-14-5, 8022-66-2
- *CHEMICAL ABSTRACTS,* 7631-86-9, 50-70-4, 77-92-9, 144-55-8, 25322-68-3, 497-19-8
- **AAS JA ; PASTER BJ ; STOKES LN et al.** *J Clin Microbiol,* 2005, vol. 43, 5721-5732
- **AINAMO J ; BARMES D ; BAEGRIC D.** *Int Dent J,* 1982, vol. 32, 281-289
- **ARABATZIS M et al.** *Br J Dermatol,* 2007, vol. 157, 681-689
- **BEREZOV AB ; DARVEAU RP.** *Periodontal 2000,* 2011, vol. 55, 36-47
- **BURNE RA ; ZENG L ; AHN SJ et al.** *Adv Dent Res,* 2012, vol. 24, 77-80
- **CALDERONE RA ; FONZI WA.** *Trends Microbiol,* 2001, vol. 9, 327-335
- **CUTTING KF ; WHITE R.** *Br J Community Nurs,* 2004, vol. 9, S6-S15
- **DARVEAU RP.** *Nat Rev Microbiol,* 2010, vol. 8, 481-490
- **DEBERNARDIS F ; LIU H ; O'MAHONY R et al.** *J Infect Dis,* 2007, vol. 195, 149-157
- **DEWHIRST FE ; CHEN T ; IZARD J et al.** *J Bacteriol,* 2010, vol. 192, 5002-5017
- **EWMA Patient Outcome Group.** *J Wound Care,* 2010, vol. 19, 239-268
- **FINDLEY K ; OH J ; YANG J et al.** *Nature,* 2013, vol. 498, 367-370
- **FRANDIN C ; KRETSCHMAR M ; NICHTERLEIN T et al.** *Mol Microbiol,* 2003, vol. 47, 1523-1543
- **GOTTRUP E ; APELQVIST J ; BJANSHOLT T et al.** *J Wound Care,* 2013, vol. 22, S1-S89
- **GOW NAR ; HUBE B.** *Curr Opin Microbiol,* 2012, vol. 15, 406-412
- **GRICE EA ; SEGRE JA.** *Annu Rev Genomics Human Genet,* 2012, vol. 13, 151-170
- **HAJISHENGALIS G ; LIANG S ; PAYNE MA et al.** *Cell Host & Microbe,* 2011, vol. 10, 497-506
- **HOLT SC ; EBERSOLE JL.** *Periodontal 2000,* 2005, vol. 38, 72-122
- **HUBE B.** *Curr Opin Microbiol,* 2004, vol. 7, 336-341
- **Human Microbiome Project Consortium.** *Nature,* 2012, vol. 486, 207-214
- **KOSTOV G ; ANGELEV M ; DENKOVA Z et al.** *Eng Life Sci,* 2011, vol. 11, 517-527
- **KOTNIK T.** *Slov Vet Res,* 2007, vol. 44 (3), 63-73
- **LANG NP ; ADLER R ; JOSS A et al.** *J Clin Periodontol,* 1990, vol. 17, 714-721
- **LIU B ; FALLER RR ; KLITGORD N et al.** *PLoS One,* 2012, vol. 7, e37919
- **MORRELL J ; STRATMAN E.** *J Agromedicine,* 2011, vol. 16 (4), 244-250
- **NAKAGAWA Y ; OHNO N ; MURAI T.** *J Infect Dis,* 2003, vol. 187, 710-713
- **OKUDA K ; KATO T ; ISHIHARA K.** *Oral Dis,* 2004, vol. 10, 5-12
- **STAPLETON AE et al.** *Clin Infect Dis,* 2011, vol. 52, 1212-1217
- **TEUGHELS W ; DURUKAN A ; OZCELIK O et al.** *J Clin Periodontol,* 2013, vol. 40, 1025-1035
- **WOUNDS UK.** *Aberdeen: Wounds UK 2010,* 2010
- **MOSKOWITZ SM ; FOSTER JM ; EMERSON J et al.** *J Clin Microbiol,* 2004, vol. 42, 1915-1920
- **BRUZUAL I ; RIGGLER P ; HADLEY S et al.** *J Antimicrob Chemother,* 2007, vol. 59, 441-450